# EUROPEAN PATENT SPECIFICATION

(11) **EP 3 169 698 B1**
(45) Date of publication and mention of the grant of the patent: **05.12.2018**
(21) Application number: 15736524.8
(22) Date of filing: 15.07.2015
(51) Int. Cl.: C07K 5/02, A61K 38/00

(54) **NOVEL COMPOUNDS**
NEUE VERBINDUNGEN
NOUVEAUX COMPOSÉS

(30) Priority: 16.07.2014 EP 14177268
(43) Date of publication of application: 24.05.2017
(73) Proprietor: The Provost, Fellows, Foundation Scholars, & the other members of Board, of the College of Holy and Undiv. Trinity of Queen Elizabeth near Dublin, 2 Dublin (IE); InvivoGen SAS, 31400 Toulouse (FR)
(72) Inventor: MILLS, Kingston, Dublin 6 (IE); KELLY, Patrick, Dublin 3 (IE); TIRABY, Jean-Gerard, 31400 Toulouse (FR); LIOUX, Thierry, 31130 Balma (FR); PEROUZEL, Eric, 31500 Toulouse (FR)
(74) Representative: Murgitroyd & Company
(86) International application number: PCT/EP2015/066203
(87) International publication number: WO 2016/008946

(56) References cited:
- WO-A1-2012/027455
- WO-A2-2007/042273
- GB-A- 2 089 816

## Description

### FIELD OF INVENTION

The present invention relates to novel NOD modulators, particularly to NOD1 or NOD1 and NOD2 agonists, processes for their preparation, pharmaceutical compositions comprising these modulators and their uses in the treatment of conditions for which agonism of NOD receptors is beneficial including inflammatory and/or autoimmune diseases, infectious or cancer diseases.

### BACKGROUND OF THE INVENTION

NOD1 and NOD2 are two members of the growing family of Nod-like receptors characterized by a nucleotide-oligomerization domain (NOD) and ligand-recognizing leucine-rich repeats. NOD1 and NOD2 are involved in the recognition of peptidoglycan (PGN), major surface component of Gram-positive bacteria. The innate immune receptors recognize specific molecules that are commonly found in microbes and induce host defense responses to eliminate invading pathogens (Creagh and O'Neill, 2006). These pathogen-recognizing receptors include membrane-bound Toll-like receptors (TLRs) as well as cytosolic NOD-like receptors and RIG-I family proteins (Inohara et al., 2005). These three classes of pathogen-recognizing receptors regulate innate and acquired immune responses by inducing intracellular signaling pathways that lead to the activation of p38, c-Jun NH2-terminal kinase (JNK), extracellular signal-regulated kinases (ERKs), caspase-1, and transcription factors including NF-κB and/or IRFs (Creagh and O'Neill, 2006). NOD1 is the founding member of the NOD-like receptor protein family that contains nucleotide oligomerization domain (NOD) and ligand-recognizing leucine-rich repeats. This family is comprised of more than 20 members including NOD2, cryopyrin, Ipaf, NAIP, and NALP1 (Creagh and O'Neill, 2006;(Inohara et al., 2005). Genetic studies have revealed that variations of the *NOD1* gene are associated with susceptibility to several human disorders including allergic diseases (Eder et al., 2006; Hysi et al., 2005; Weidinger et al., 2005), Crohn's disease (McGovern et al., 2005), and sarcoidosis (Tanabe et al., 2006), although the mechanism underlying these disease associations remains poorly understood. Previous studies suggested the involvement of NOD1 in the recognition of numerous bacteria including *Helicobacter pylori* (Boughan et al., 2006; Viala et al., 2004), *Listeria monocytogenes* (Opitz et al., 2006; Park et al., 2007), *Shigella flexneri* (Girardin et al., 2001), several *Bacillus* species (Hasegawa et al., 2006), and *Propionibacterium acnes* (Tanabe et al., 2006). NOD1 was expressed in multiple tissues and is thought to have an important role, particularly, in epithelium (Inohara et al., 1999). It has been suggested that NOD1 plays a role in intestinal immunity by regulating innate immune responses of epithelial cells infected with invasive enterobacteria and *Helicobacter pylori* (Kim et al., 2004; Viala et al., 2004).

Several NOD1 polymorphisms are linked to the development of inflammatory bowel disease, asthma and atopic eczema (Huebner et al., 2009; Hysi et al., 2005; McGovern et al., 2005; Weidinger et al., 2005). The NOD1 agonists are potentially useful as vaccine adjuvants and to prevent, inhibit or treat a variety of disorders including bowel diseases, asthma and cancer.

US patent applications 2006/0194740 A1 and 2009/0028795 A1 provide compositions and methods for treating tumors that involve increasing the expression of NOD1 and/or the activity of NOD1. US patent 7244557 B2 provides a method for screening modulators of NOD1 signaling. US patent 7396812 B2 provides a method for modulating NOD1 activity, use of a MTP related molecule for modulating NOD1 activity, and therapeutic applications thereof.

There is evidence that NOD1 mediates host recognition of bacteria or bacterial fractions containing soluble PGN-related molecules with the essential iE-DAP dipeptide (Girardin et al., 2001; Inohara et al., 2001). NOD1 senses the essential iE-DAP dipeptide, which is uniquely found in PGN of all Gram-negative and certain Gram-positive bacteria (Chamaillard et al., 2003; Girardin et al., 2003a; Girardin et al., 2003b; Girardin et al., 2003c; Inohara et al., 2003), whereas NOD2 recognizes the muramyl dipeptide (MDP) structure in bacterial peptidoglycan (PGN)-related molecules. Although the iE-DAP structure is found in the insoluble fraction of intact PGN, intermediates of PGN synthesis and cleaved PGN products produced during bacterial growth and PGN recycling (Girardin et al., 2003b; Holtje, 1998), the identity of the major NOD1 stimulatory molecule (s) remains unknown (18). Previous studies have shown that several iE-DAP-containing molecules including iE-DAP, D-Ala-γ-Glu-*meso*-DAP (A-iEDAP), and GlcNAc-anhMurNAc-D-Ala-γ-Glu-*meso-*DAP, but not intact macromolecular PGN, stimulate NOD1 (Chamaillard et al., 2003).

WO2010/072797 discloses that the NOD1 agonist analogs can have immunosuppressive activity and can act to selectively suppress Th1 and Th17 (IL-17 producing T cell) T cell mediated inflammatory autoimmune disease. It has been identified that the in-vivo administration of TriDAP involves an anti-inflammatory effect.

US Patent N°4,401,658 discloses tri-tetra or penta pepdide, comprising L-Ala-D-Glu residues and their use as vaccine adjuvants and immunostimulants. Particularly, they are useful to increase the hypersensitivity reactions and/or the production of circulating antibodies against the antigens with which they are administered, and they can stimulate, defence reactions against certain infections.

WO2012/027455 discloses certain glutathione lanthionine compounds and their use in the treatment of inflammatory diseases, CNS diseases selected from the group consisting of: sepsis; amyotrophic lateral sclerosis (ALS), a degenerative motor neuron disease, Alzheimer's disease, Parkinson's disease, Huntington's disease, multiple sclerosis, macular degeneration, cardiovascular diseases, atherosclerosis, rheumatoid arthritis; lupus; fibromyalgia; neuropathy; allergy; autoimmune disease; diabetes; ulcerative colitis; or inflammatory bowel disease (IBD) hypertension, attention deficit disorder, depression (e.g., major depression); schizophrenia; chronic pain and generalized anxiety disorders.

In light of the role NODs plays in the pathogenesis of diseases, it is desirable to prepare compounds that modulate NODs activity and hence have utility in the treatment of diseases mediated by NODs such as inflammatory and/or autoimmune diseases, infectious and cancer diseases.

### SUMMARY OF THE INVENTION

The present invention provides novel NOD modulators, specifically NOD1 or NOD1 and NOD2 agonists, processes for their preparation, pharmaceutical compositions comprising these modulators and their uses in the treatment of conditions for which agonism of NOD receptors is beneficial including inflammatory and/or autoimmune diseases, infectious and cancer diseases.

More specifically, the present invention is directed to a compound or a pharmaceutically acceptable salt thereof, selected from 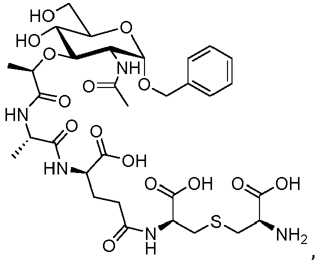 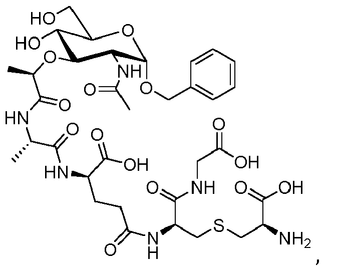 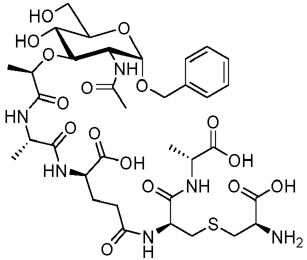 (herein after compounds of the invention)
or a pharmaceutically acceptable salt thereof.

In one aspect, the present invention provides a pharmaceutical composition comprising a) a compound of the invention or a pharmaceutically acceptable salt thereof and b) one or more pharmaceutically acceptable excipients.

In a further aspect, the present invention provides a compound of the invention , or a pharmaceutically acceptable salt thereof, for use in therapy.

Compounds of the invention and pharmaceutically acceptable salts thereof, are modulators of NOD, particularly they are agonists of NOD1 or NOD1 and NOD2 and can be useful in the treatment of inflammatory and/or autoimmune diseases mediated by NOD receptors.

Compounds of the invention and pharmaceutically acceptable salts thereof can be useful in the treatment of a variety of cancers and tumours.

Furthermore the compounds of the invention and pharmaceutically acceptable salts thereof can be useful in the treatment of infectious diseases particularly in preventing or treating Gram-negative bacteria infection.

In a further aspect, the present invention provides a compound of the invention or a pharmaceutically acceptable salt thereof, for use in treatment of conditions for which agonism of NOD1 or NODland NOD2 receptors is beneficial.

In a further aspect, the present invention provides a compound of the invention, or a pharmaceutically acceptable salt thereof, for use in treatment of inflammatory and or autoimmune diseases, cancers and tumours and infectious diseases.

In yet a further aspect, the present invention is directed to method of treating inflammatory and/or autoimmune diseases, cancers and tumours and infectious diseases, which comprises administering to a subject in need thereof a therapeutically amount of a compound of the invention or a pharmaceutically acceptable salt thereof.

### BRIEF DESCRIPTION OF THE DRAWINGS

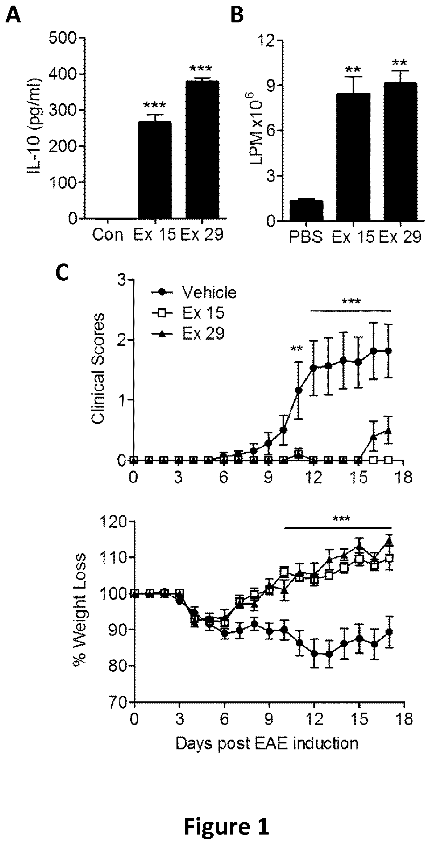

**Figure 1C** shows Experimental Autoimmune Encephalomyelitis (EAE) clinical scores and percentage weight loss.

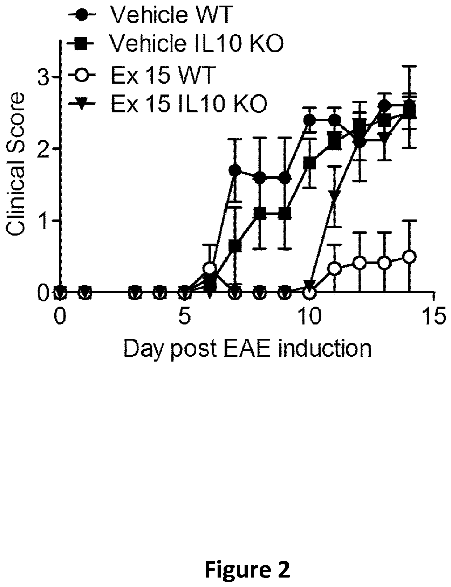

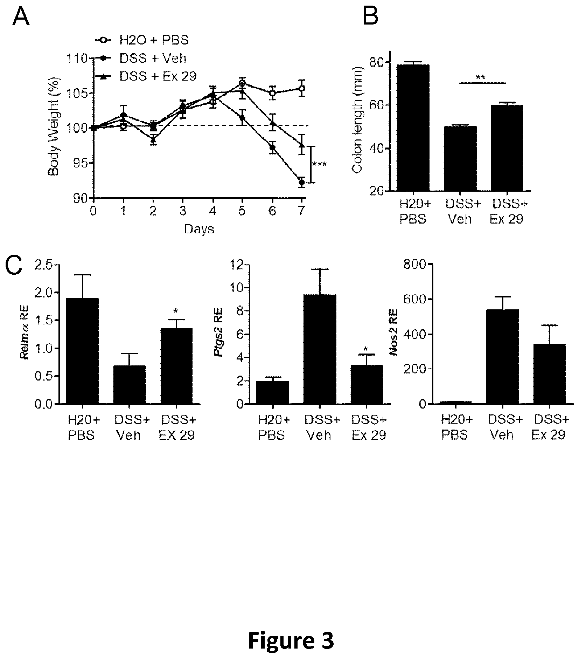

**Figure 2** shows the role of IL-10 in protection against EAE induced by NOD1 activation.

### DETAILED DESCRIPTION OF THE INVENTION

In a first aspect, the present invention is directed to compounds selected from 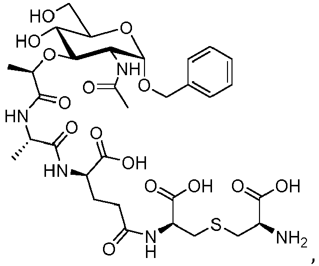 *N²*-(((*R*)-2-(((*2S,3R,4R,5S,6R*)-3-acetamido-2-(benzyloxy)-5-hydroxy-6-(hydroxymethyl)tetrahydro-*2H*-pyran-4-yl)oxy)propanoyl)-*L*-alanyl)-*N⁵*-((*S*)-2-(((*R*)-2-amino-2-carboxyethyl)thio)-1-carboxyethyl)-*D*-glutamine; 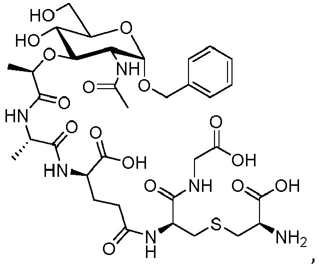 *N²*-(((*R*)-2-(((*2S,3R,4R,5S,6R*)-3-acetamido-2-(benzyloxy)-5-hydroxy-6-(hydroxymethyl)tetrahydro-*2H*-pyran-4-yl)oxy)propanoyl)-*L*-alanyl)-*N⁵*-((*S*)-3-(((*R*)-2-amino-2-carboxyethyl)thio)-1-((carboxymethyl)amino)-1-oxopropan-2-yl)-*D*-glutamine; 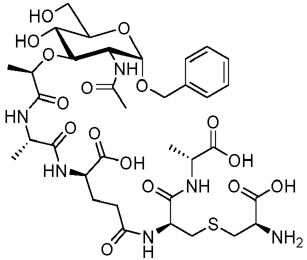 *N₂*-(((*R*)-2-(((*2S,3R,4R,5S,6R*)-3-acetamido-2-(benzyloxy)-5-hydroxy-6-(hydroxymethyl)tetrahydro-2H-pyran-4-yl)oxy)propanoyl)-L-alanyl)-*N⁵*-((*S*)-3-(((*R*)-2-amino-2-carboxyethyl)thio)-1-(((*R*)-1-carboxyethyl)amino)-1-oxopropan-2-yl)-D-glutamine;
or a pharmaceutically acceptable salt thereof.

Compounds according of the invention contain a basic functional group and are therefore capable of forming pharmaceutically acceptable acid addition salts by treatment with a suitable acid.

Suitable acids include pharmaceutically acceptable inorganic acids and pharmaceutically acceptable organic acids.

Representative pharmaceutically acceptable acid addition salts include hydrochloride, hydrobromide, nitrate, methylnitrate, sulfate, bisulfate, sulfamate, phosphate, acetate, hydroxyacetate, phenylacetate, propionate, butyrate, isobutyrate, valerate, maleate, hydroxymaleate, acrylate, fumarate, malate, tartrate, citrate, salicylate, p-aminosalicyclate, glycollate, lactate, heptanoate, phthalate, oxalate, succinate, benzoate, o-acetoxybenzoate, chlorobenzoate, methylbenzoate, dinitrobenzoate, hydroxybenzoate, methoxybenzoate, naphthoate, hydroxynaphthoate, mandelate, tannate, formate, stearate, ascorbate, palmitate, oleate, pyruvate, pamoate, malonate, laurate, glutarate, glutamate, estolate, methanesulfonate (mesylate), ethanesulfonate (esylate), 2- hydroxyethanesulfonate, benzenesulfonate (besylate), p-aminobenzenesulfonate, p- toluenesulfonate (tosylate), and napthalene-2-sulfonate.

In certain embodiments, compounds according to the invention may contain an acidic functional group. Suitable pharmaceutically acceptable salts include salts of such acidic functional groups.

Representative salts include pharmaceutically acceptable metal salts such as sodium, potassium, lithium, calcium, magnesium, aluminium, and zinc salts; carbonates and bicarbonates of a pharmaceutically acceptable metal cation such as sodium, potassium, lithium, calcium, magnesium, aluminium, and zinc; pharmaceutically acceptable organic primary, secondary, and tertiary amines including aliphatic amines, aromatic amines, aliphatic diamines, and hydroxy alkylamines such as methylamine, ethylamine, 2-hydroxyethylamine, diethylamine, triethylamine, ethylenediamine, ethanolamine, diethanolamine, and cyclohexylamine.

For reviews on suitable pharmaceutical salts see Berge et al, J. Pharm, Sci., 66, 1-19, 1977; P L Gould, International Journal of Pharmaceutics, 33 (1986), 201-217; and Bighley et al, Encyclopaedia of Pharmaceutical Technology, Marcel Dekker Inc, New York 1996, Volume 13, page 453-497.

Other salts that are not deemed pharmaceutically acceptable may be useful in the preparation of compounds of the invention and are included within the scope of the invention, such as those formed with ammonia and trifluoroacetic acid.

The present invention encompasses all possible stoichiometric and non-stoichiometric forms of the salts of the compounds of the invention.

These pharmaceutically acceptable salts may be prepared in situ during the final isolation and purification of the compound, or separately, by reacting the purified compound in its free acid or free base form with a suitable base or acid, respectively.

The person skilled in the art will appreciate that many organic compounds can form complexes with solvents in which they are reacted or from which they are precipitated or crystallised. These complexes are known as "solvates". Where the solvent is water the complex is known as a "hydrate".

The present invention encompasses all solvates of the compounds of the invention . In addition, prodrugs are also included within the context of this invention.

Prodrugs are any covalently bonded carriers that release a compound of formula (I) in vivo when such prodrug is administered to a patient. Prodrugs are generally prepared by modifying functional groups in a way such that the modification is cleaved, either by routine manipulation or in vivo, yielding the parent compound. Prodrugs include, for example, compounds of this invention wherein hydroxy, amine or sulfhydryl groups are bonded to any group that, when administered to a patient, cleaves to form the hydroxy, amine or sulfhydryl groups. Thus, representative examples of prodrugs include (but are not limited to) acetate, formate and benzoate derivatives of alcohol, sulfhydryl and amine functional groups of the compounds of formula (I). Further, in the case of a carboxylic acid (-COOH), esters may be employed, such as methyl esters, ethyl esters, and the like. Esters may be active in their own right and /or be hydrolysable under in vivo conditions in the human body. Suitable pharmaceutically acceptable in vivo hydrolysable ester groups include those which break down readily in the human body to leave the parent acid or its salt.

The compounds of the invention and pharmaceutically acceptable salts thereof contain at least three asymmetric centers (also referred to as a chiral center) and may, therefore, exist as individual enantiomers, diastereomers, or other stereoisomeric forms, or as mixtures thereof.

Chiral centers, such as chiral carbon atoms, may also be present in a substituent such as an alkyl group. Where the stereochemistry of a chiral center present in a compound of the invention, or in any chemical structure illustrated herein, is not specified the structure is intended to encompass all individual stereoisomers and all mixtures thereof. Thus, compounds of the invention and pharmaceutically acceptable salts thereof may be used as racemic mixtures, enantiomerically enriched mixtures, or as enantiomerically pure individual stereoisomers.

Included within the scope of the "compounds of the invention" are all solvates, hydrates, prodrugs, radiolabelled derivatives.

The invention also includes isotopically-labeled compounds, which are identical to those described herein, but for the fact that one or more atoms are replaced by an atom having an atomic mass or mass number different from the atomic mass or mass number usually found in nature. Examples of isotopes that can be incorporated into compounds of the invention include isotopes of hydrogen, carbon, nitrogen, oxygen, phosphorous, fluorine, iodine, and chlorine, such as ³H, ¹¹C, ¹⁴C, ¹⁸F, ¹²³I and ¹²⁵I. Compounds of the invention that contain the aforementioned isotopes and/or other isotopes of other atoms are within the scope of the present invention. Isotopically-labeled compounds of the present invention, for example those into which radioactive isotopes such as ³H, ¹⁴C are incorporated, are useful in drug and/or substrate tissue distribution assays. Tritiated, i.e., ³H, and carbon-14, i.e., ¹⁴C, isotopes are particularly preferred for their ease of preparation and detectability. ¹¹C and ⁸F isotopes are particularly useful in PET (positron emission tomography), and ¹²⁵I isotopes are particularly useful in SPECT (single photon emission computerized tomography), all useful in brain imaging. Further, substitution with heavier isotopes such as deuterium, i.e., ²H, can afford certain therapeutic advantages resulting from greater metabolic stability, for example increased *in vivo* half-life or reduced dosage requirements and, hence, may be preferred in some circumstances.

Isotopically labeled compounds of the invention can generally be prepared by carrying out the procedures disclosed in the Examples below, then substituting a readily available isotopically labeled reagent for a non-isotopically labeled reagent.

### DEFINITIONS

Throughout the present specification and the accompanying claims the words "comprise" and "include" and variations such as "comprises", "comprising", "includes" and "including" are to be interpreted inclusively. That is, these words are intended to convey the possible inclusion of other elements or integers not specifically recited, where the context allows.

A peptide is an amino acid polymer wherein each amino acid is linked to its neighbor by an amide bond -CO-NH-, also called peptide bond. The number of amino acids contained in one peptide can vary from 2 to about 100, though the latter is not set precisely. Typically, both extremities of the peptide backbone finish with an amino group on one side and a carboxylic acid group on the other side. The amine extremity is referred to as the "N-terminal extremity" and the carboxylic acid extremity is referred to as the "C-terminal extremity".

As used herein, the term "salt" refers to any salt of a compound according to the present invention prepared from an inorganic or organic acid or base, quaternary ammonium salts and internally formed salts.

As used herein, the term "pharmaceutically acceptable salts" refers to salts that retain the desired biological activity of the subject compound and exhibit minimal undesired toxicological effects.

As used herein, the term "prodrug" means a compound which is converted within the body, e.g. by hydrolysis in the blood, into its active form that has medical effects. Pharmaceutically acceptable prodrugs are described in T. Higuchi and V. Stella, Prodrugs as Novel Delivery Systems, Vol. 14 of the A.C.S. Symposium Series, Edward B. Roche, ed., Bioreversible Carriers in Drug Design, American Pharmaceutical Association and Pergamon Press, 1987, and in D. Fleisher, S. Ramon and H. Barbra "Improved oral drug delivery: solubility limitations overcome by the use of prodrugs", Advanced Drug Delivery Reviews (1996) 19(2) 115-130, each of which are incorporated herein by reference.

As used herein, the term "solvate" refers to a complex of variable stoichiometry formed by a solute (in this invention, a compound of formula (I) or a salt thereof) and a solvent. Such solvents for the purpose of the invention may not interfere with the biological activity of the solute. Examples of suitable solvents include, but are not limited to, water, methanol, ethanol and acetic acid. In one embodiment, the solvent used is a pharmaceutically acceptable solvent. Examples of suitable pharmaceutically acceptable solvents include water, ethanol and acetic acid. In one embodiment, the solvent used is water.

Unless otherwise specified, the term "residue" with reference to an amino acid or amino acid derivative means a radical derived from the corresponding α-amino acid by eliminating the hydroxyl of the C-terminal extremity and/or one hydrogen of the N-terminal extremity. For instance, the terms Ala, Gly, Ile, Glu, Phe, Ser, Leu, Cys, represent the "residues" of L-alanine, glycine, L-isoleucine, L-glutamic acid, L-phenylalanine, L-serine, L-leucine, and L-cysteine, respectively. The term alanyl or glycyl residue refers to radical derived from the corresponding α-amino acid by eliminating one hydrogen of the N-terminal extremity.

The term "side chain" with reference to an amino acid or amino acid residue means a group attached to the α-carbon atom of the α-amino acid. For example, the R-group side chain for glycine is hydrogen, for alanine it is methyl, for valine it is isopropyl. For the specific R-groups or side chains of the α-amino acids reference is made to A.L. Lehninger's text on Biochemistry (see chapter 4).

The term "chiral" refers to molecules which have the property of non-superimposability on their mirror image partner.

The term "chiral carbon atom" or "chiral center" is a carbon atom which has four different substituents.

The lanthionine(VI) has two chiral centers showed in the structure as *and it can exists in 3 diastereoisomers 2 *threo* (LL and DD) and 1 *meso* (DL or LD).

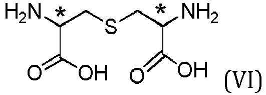

The term amino or amine refers to -NH2 group.

The term carboxy or carboxyl refers to -COOH group.

The term hydroxy or hydroxyl refer to -OH group.

The term "protecting group" as used herein means temporary substituents which protect a potentially reactive functional group from undesired chemical transformations. Examples of such protecting groups include esters of carboxylic acids, carbamyl of amines respectively. The field of protecting group chemistry has been reviewed (Greene, T.W.; Wuts, P.G.M. Protective Groups in Organic Synthesis, 2nd ed.; Wiley: New York, 1991).

As used herein, the term "treatment" refers to prophylaxis of the condition, ameliorating or stabilising the specified condition, reducing or eliminating the symptoms of the condition, slowing or eliminating the progression of the condition, and preventing or delaying reoccurrence of the condition in a previously afflicted patient or subject.

As used herein, the term "therapeutically effective amount" refers to the quantity of a compound of formula (I), or a pharmaceutically acceptable salt thereof, which will elicit the desired biological response in an animal or human body.

As used herein, the term "subject" refers to an animal or human body.

The terms "D amino acid" or "D" and "L amino acid"or "L" as used herein refer to absolute configuration of the amino acid, rather than a particular direction of rotation of plane-polarized light. The usage herein is consistent with standard usage by those of skill in the art.

Thus, "D" or "L" refers to absolute configuration based on the assignment of the absolute configuration of the dextrotatory and levorotatory forms of glyceraldehyde.

The terms "solid support", "solid phase" and "resin", refer indifferently in the present invention to a support conventionally used in organic chemistry, and particularly in peptide synthesis comprising a matrix polymer and a linker.

The solid support comprises a base matrix such as gelatinous or macroporous resins, for example polystyrene, and an anchor referred to as a "linker" designed to produce after cleavage a given functionality. The name of the solid support, in all strictness, refers to the name of the linker and the matrix.

Advantageously the base support is chosen among the polystyrene supports, polyamide supports, polyethylene glycol supports, polyacrylic supports, composite supports and copolymers thereof, it being possible for said support to be in the form of beads, of a film-coated support such as rings or lanterns, of a plug, or of a non-cross-linked soluble support.

More advantageously, the base support is selected from gelatinous or macroporous resins having a matrix with polystyrene (PS), or having a matrix with polyamide (PL) or polyethylene glycol (PEG), or else composite supports of polyethylene glycol-polystyrene (PEG-PS) or polyethylene glycol-dimethylacrylamide (PEGA).

Advantageously, the solid support is chosen in the group comprising acid-labile resins. Among linkers, TFA-labile ones are especially useful for the production of combinatorial libraries. Indeed, by using methodology cleavage and post-cleavage, workups are straightforward and often require only simple evaporation of TFA that can be done in parallel using vacuum centrifugator or inert gas bubbling.

The present invention also provides processes for the preparation of a compound of the invention or a pharmaceutically acceptable salt thereof.

Thus, compounds of the invention may be prepared by methods known in the art of organic synthesis, particularly in the peptide synthesis as set forth in part in the following synthesis schemes.

The methodology involves the formation of peptide bond by any well known procedure in the art, such as solid phase synthesis or liquid phase synthesis.

The most commonly employed methods for peptide bond formation in solution include: N,N'-dicyclohexylcarbodiimide (DCC) or 1-ethyl-3-(3'-dimethylaminopropyl)carbodiimide hydrochloride (EDC), i.e. water-soluble carbodiimide, O-(1 H-benzotriazol- 1 -yl)-N,N,N',N'-tetramethyluronium tetrafluoroborate (TBTU), benzotriazol-l-yl-oxytris(dimethylamino)phosphonium hexafluorophosphate (BOP), benzotriazole-1-yl-oxy-tris-pyrrolidino-phosphonium hexafluorophosphate (PyBOP), O-(7-azabenzotriazol- 1 -yl)- 1 ,2,3-tetramethyluronium hexafluorophosphate (HATU), O-benzotriazol- 1 -yl-N,N,N',N'-tetramethyluronium hexafluorophosphate (HBTU), O-benzotriazol- 1 -yl-tetramethyltetrafluoroborate (TBTU), N-hydroxy- 5-norbornene-2,3-dicarbodiimide, or any other coupling agent in a solvent such as ether, acetone, chloroform, dichloromethane, ethyl acetate, DMF, tetrahydrofuran (THF), acetonitrile, dimethylsulfoxide (DMSO), N-methyl pyrrolidinone (NMP), under ice-cooling or at room temperature, preferably in the presence of an acylation catalyst such as dimethylaminopyridine (DMAP), pyridine, N-hydroxybenzotriazole (HOBt), l-hydroxy-7-azabenzotriazole (HOAt), N-hydroxy succinimide and the like.

Preferably the coupling reaction is carried out by employing PyBOP or HATU in an organic solvent such as DCM and/or DMF in presence of a base like NMM (N-methyl imidazole) or DIEA (diisopropyl ethylamine).

PyBOP or HATU react exclusively with carboxylate salts (R-COO-); mixtures of PyBOP or HATU and a carboxylic acid (R-COOH) remain stable. This procedure eliminates the requirement for a separate neutralization step saving time and minimizing diketopiperazine formation. Three equivalents of base (DIEA or NMM) are necessary to neutralize the carboxylic acid, the amine salt, and the acidic hydroxybenzotriazole or 7-azabenzotriazole.

When using PyBOP or HATU, the reaction mixture has to be kept near basic pH in order to ensure a fast coupling. Under such conditions, the coupling rate is so high that racemization is negligible using urethane-protected amino acid couplings and fairly low in segment coupling. The excess of acid and "onium" salt (PyBOP or HATU) is typically 1.1 molar equivalent in solution synthesis. In solid phase, excess from 1.5-3 equivalents are commonly used. The by-products are water and DCM soluble that allows purification of product by precipitation by water or diethyl ether (in solution synthesis) or purification by flash chromatography.

This reaction is preferably carried out in a solvent such as methylene chloride, chloroform, tetrahydrofuran, dioxane, dimethylformamide ethyl acetate, or the like, under ice-cooling or at ambient temperature, and the reaction in the presence of an inert gas is usually carried out in anhydrous conditions.

In the synthesis of the compounds of the invention it will be necessary, or at least desirable, in many reactions to protect the amino groups and/or the carboxy groups. The synthetic route chosen for the di, tri, tetra or pentapeptide synthesis may require removal of one or the other or both of said protecting groups in order to permit further reaction at the regenerated amino or carboxy group; i.e., the protecting groups used are reversible and, in most instances, are removable independently of each other. Additionally, the choice of protecting group for a given amino group depends upon the role of said amino group in the overall reaction scheme. Amino protecting groups having varying levels of lability, i.e., ease of removal, will be used. The same is true as regards carboxy protecting groups. Such groups are known in the art and attention is directed to the reviews by Bodansky et al., "Peptide Synthesis", 2nd Ed., John Wiley & Sons, N.Y. (1976).

Examples of conventional amino protecting groups include vinyl, allyl, t-butoxycarbonyl, benzyl, benzyloxycarbonyl, 2-nitrobenzyl, 4-nitrobenzyl, 2-nitrobenzyloxylcarbonyl, formyl, benzoyl, acetyl, ethylcarbonyl, chloroacetyl, trichloroacetyl, trifluoroacetyl, methyloxycarbonyl, allyloxycarbonyl, trimethylsilyl, triethylsilyl, triphenylsilyl, t-butyl-dimethylsilyl, methyldiphenylsilyl, 2,4-dimethoxybenzyl, 2,4-dimethoxybenzyloxycarbonyl, 4-methoxybenzyl, 4-(methoxymethyloxy)phenyl, bis-(4-methoxyphenyl)methyl, t-butoxycarbonylmethyl, allyoxycarbonylmethyl, methoxymethyl, methylthiomethyl, methoxyethoxymethyl, [2-(trimethylsilyl)ethoxy]methyl or 2-(methylthiomethoxy)ethoxycarbonyl. In general, amino protecting groups which are readily removed under acid conditions or catalytic hydrogenolysis are preferred, e.g. t-butoxycarbonyl, benzyloxycarbonyl and triphenylmethyl.

Conventional carboxy-protecting groups which can be employed in the present invention to block or protect the carboxylic acid function are well-known to those skilled in the art and, preferably, said groups can be removed, if desired, by methods which do not result in any appreciable destruction of the remaining portion of the molecule, for example, by chemical or enzymatic hydrolysis, treatment with chemical reducing agents under mild conditions, irradiation with ultraviolet light or catalytic hydrogenation. Examples of such readily removable carboxy-protecting groups include moieties such as C₁-C₆ alkyl, diphenylmethyl (benzyhydryl), 2-naphthylmethyl, 4-pyridylmethyl, phenacyl, acetonyl, 2,2,2-trichloroethyl, silyl such as trimethylsilyl and t-butyldimethylsilyl, phenyl, ring substituted phenyl, e.g., 4-chlorophenyl, tolyl, and t-butylphenyl, phenyl C1-6 alkyl, ring substituted phenyl C1-6 alkyl, e.g., benzyl, 4-methoxybenzyl, 4-nitrobenzyl (p-nitrobenzyl), 2-nitrobenzyl (o-nitrobenzyl), and triphenylmethyl (trityl), methoxymethyl, 2,2,2-trichloroethoxycarbonyl, benzyloxymethyl, C₁-C₆ alkanoyloxy C₁-C₆ alkyl such as acetoxymethyl, propionyloxymethyl, C₂-C₆ alkenyl such as vinyl and allyl. Particularly advantageous carboxy protecting groups are benzyl, p-nitrobenzyl, o-nitrobenzyl, 2,4-dimethoxybenzyl, 4-methoxybenzyl, allyl, substituted allyl, t-butyl or diphenylmethyl (DMP).

Other suitable amino and carboxy-protecting groups well known to those skilled in the art can be found in "Protective Groups in Organic Synthesis", 3rd Ed. Theodora W. Greene (John Wiley & Sons, 1999), incorporated herein by reference.

After synthesis of the desired peptide, it is subjected to the deprotection reaction and, in the case of solid peptide synthesis, cut out from the solid support. Such peptide cutting reaction can be carried with hydrogen fluoride or trifluoromethane sulfonic acid for the Boc method, and with TFA for the Fmoc method.

The deprotection method used during stepwise assembly of peptide chain and for the final deblocking of all functional groups to yield the free peptide is determined by the synthetic strategy. In the present invention, suitable orthogonal amine protecting groups may be selected and deprotection may be affected for the group using conditions amenable to hydrolysis of the group.

For example, protective groups can be removed by acid, base, and hydrogenolysis. Groups such as trityl, dimethoxytrityl, acetal and t-butyldimethylsilyl are acid labile and may be used to protect carboxy and hydroxy reactive moieties in the presence of amino groups protected with Cbz groups, which are removable by hydrogenolysis, and Fmoc groups, which are base labile. Carboxylic acid and hydroxy reactive moieties may be blocked with base labile groups such as, without limitation, methyl, ethyl, and acetyl in the presence of amines blocked with acid labile groups such as t-butyl carbamate or with carbamates that are both acid and base stable but hydrolytically removable.

Carboxylic acid and hydroxy reactive moieties may also be blocked with hydrolytically removable protective groups such as the benzyl group, while amine groups capable of hydrogen bonding with acids may be blocked with base labile groups such as Fmoc. Carboxylic acid reactive moieties may be blocked with oxidative ly-removable protective groups such as 2,4-dimethoxybenzyl, while coexisting amino groups may be blocked with fluoride labile silyl carbamates.

Allyl blocking groups are useful in the presence of acid- and base- protecting groups since the former are stable and can be subsequently removed by metal or pi-acid catalysts. For example, an allyl-blocked carboxylic acid can be deprotected with a palladium (0)-catalyzed reaction in the presence of acid labile t-butyl carbamate or base- labile acetate amine protecting groups. Yet another form of protecting group is a resin to which a compound or intermediate may be attached. As long as the residue is attached to the resin, that functional group is blocked and cannot react. Once released from the resin, the functional group is available to react.

For the final deprotection, several options for deprotection reaction processes are shown below and include (a) catalytic hydrogenolysis using molecular hydrogen and palladium (H₂/Pd) or catalytic transfer hydrogenolysis; (b) reduction with metallic sodium in liquid ammonia and (c) or reaction with strong acids using halogenated acids or hydrohalogenic acids, e.g. hydrobromic acid in acetic acid (HBr/AcOH) or liquid HF, BBr₃/DCM, TFA/thioanisole and sulfonic acids.

Of these, cleavage of protecting groups under neutral conditions is highly desirable because such procedures would:
(a) be free from racemization;
(b) leave acid sensitive amino acids intact;
(c) be useful particularly in the synthesis of acid/base sensitive biologically active peptides; and
(d) eliminate side reactions that are commonly observed in acidolysis and base treatments.

Two such methods, catalytic hydrogenation (Schlatter, et al. 1977; Rylander, 1979).

In Hydrogenation and Hydrogenolysis in Synthetic Organic Chemistry, Delft University Press, 1977) and catalytic transfer hydrogenation (Khan & Sivanandaiah, 1978; Anwer & Spatola, 1981; Anantharamaiah & Sivanandaiah, 1977) have become popular for the cleavage of benzyl esters, benzyl ethers and benzyloxycarbonyl protecting groups.

The only limitation being the intolerance of palladium catalysts to the presence of divalent sulphur. While hydrogenation with gaseous hydrogen is typically performed under high pressure (Parr apparatus), transfer hydrogenation is typically performed under ambient pressure. The transfer hydrogenation procedure utilizes simple organic molecules (cyclohexene, cyclohexadiene, formic acid) or inorganic compounds (phosphinic acid and its salts, hydrazine) as an in situ source of hydrogen.

In the examples presented herein, certain protecting and activating groups are specifically illustrated. However, one skilled in the art will recognize that other protecting or activating groups could have been used. The choice of a particular protecting group is dependent to a great extent upon the availability of the necessary reagent, its effect upon solubility of the "protected" compound, its ease of removal and the presence of other groups which might be effected by its use; i.e. its selectivity, or its removal.

"S-protecting group" means a substituent such as the S-protecting groups disclosed in Greene, "Protective Groups in Organic Synthesis", Wiley, New York, 2007 4th edition Paul Lloyd- Williams, Fernando Albericio, Ernest Giralt, "Chemical Approaches to the Synthesis of Peptides and Proteins", CRC Press, 1997 or Houben-Weyl, "Methods of Organic Chemistry, Synthesis of Peptides and Peptidomimetics", Vol E 22a, Vol E 22b, Vol E 22c, Vol E 22d., M. Goodmann Ed., Georg Thieme Verlag, 2002, which protects reactive sulphur against undesirable reactions.

"S-protecting group" can comprise the benzyl group, a Trt group, acetamidomethyl, the S-tert-butyl, S-acetyl, S-methoxymethyl, as the protecting group for a free mercapto group in the amino acid residue and the like.

During the preparation of compounds of the present invention, or intermediates thereto, it may also be desirable or necessary to prevent cross-reaction between chemically active substituents other than those present on naturally occurring or other amino acids. The substituents may be protected by standard blocking groups which may subsequently be removed or retained, as required, by known methods to afford the desired products or intermediates. Selective protection or deprotection may also be necessary or desirable to allow conversion or removal of existing substituents, or to allow subsequent reaction to afford the final desired product.

The selection of a suitable protecting group depends upon the functional group being protected, the conditions to which the protecting group is being exposed and to other functional groups which may be present in the molecule.

The compounds of this invention can be prepared by chemical synthetic methods details of which will be apparent from the following description.

The crude compounds or intermediates obtained according to the processes described herein can be subjected to purification. Purification is carried out by any one of the methods known for this purpose, i.e. any conventional procedure involving extraction, precipitation, chromatography, electrophoresis, or the like. For example, HPLC (high performance liquid chromatography) can be used. The elution can be carried using a water-acetonitrile-based solvent commonly employed for protein purification.

Another object of the present invention relates to a process for producing a solid phase peptide of compounds of the invention.

Solid phase peptide synthesis techniques may follow any methods conventionally employed for peptide synthesis, for example, employing a condensing agent method, an azide method, a chloride method, an acid anhydride method, a mixed anhydride method, an active ester method, an enzyme method as disclosed in Nobuo Izumiya et al., "Fundamentals and Experiments for Peptide Synthesis (in Japanese)", issued from Maruzen Co., Ltd., 1985, or a standard Fmoc protocol, see e.g. Carpino et al., 1970, J. Am. Chem. Soc. 92(19):5748-5749; Carpino et al., 1972, J. Org. Chem. 37(22):3404-3409.

The process consists in coupling, an aminoacid or a peptide of the general formula (XXII), 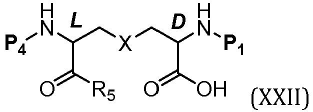 in which Rs is hydroxyl, D-alanyl or a glycyl residue and X is sulphur have the meanings of formula(I) or they are protecting groups thereof, P₁ and P₄ represent a nitrogen protecting group, using the general methods described above for obtaining a peptide bond, followed by removal of any protecting group and where P₁ and P₄ are orthogonal protections. with an activated solid support of the following formula (XXIII): 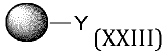 wherein: 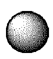 is a solid support as defined above, and Y represents an activating group comprising chlorides, bromides, iodides, p-nitrophenolate.

The coupling reaction may be carried out by employing a condensing reagent such as N,N'-dicyclohexylcarbodiimide (DCC) or 1-ethyl-3-(3'-dimethylaminopropyl)carbodiimide hydrochloride (EDC), i.e. water-soluble carbodiimide, O-(1 H-benzotriazol-1-yl)-N,N,N',N'-tetramethyluronium tetrafluoroborate (TBTU), benzotriazol-1-yl-oxytris(dimethylamino)phosphonium hexafluorophosphate (BOP), O-(7-azabenzotriazol-1-yl)-1,2,3-tetramethyluronium hexafluorophosphate (HATU), O-benzotriazol-1-yl-N,N,N',N'-tetramethyluronium hexafluorophosphate (HBTU), O-benzotriazol-1-yl-tetramethyltetrafluoroborate (TBTU), N-hydroxy- 5-norbornene-2,3-dicarbodiimide, or any other coupling agent in a solvent such as ether, acetone, chloroform, dichloromethane, ethyl acetate, DMF, tetrahydrofuran (THF), acetonitrile, dimethylsulfoxide (DMSO), N-methyl pyrrolidinone (NMP), under ice-cooling or at room temperature, preferably in the presence of an acylation catalyst such as dimethylaminopyridine (DMAP), pyridine, N-hydroxybenzotriazole (HOBt), l-hydroxy-7-azabenzotriazole (HOAt), N-hydroxy succinimide and the like.

Preferably the coupling reaction is carried out by employing DCC, DMAP in an organic solvent such as DCM and/or DMF.

It being understood that the protective groups of the amine group, and the condition of cleavage of the solid support, are different. Following to removal of the amine group protecting group P₄ the compound so obtained can be condensed with compound of formula (XXIV) 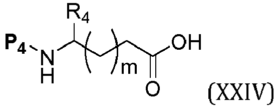 in which m is 1 and R₄ is COOH or a protected derivative thereof and P₄ represent a nitrogen protecting group.

The product so obtained by removal of nitrogen protecting group P₄ can be reacted with an activated derivative of the general formula (XXV) to obtain a compound of of the invention. 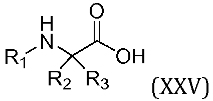 in which R₁, is Benzyl-2-acetamido-4,6-0-dibenzylidene-2-deoxy-3-0-[(R)-1-carboxyethyl]-α-D-glucopyranoside are hydrogen Following the removal of the amine group protected by R₁, the product so obtained can be reacted with an activated derivative of the general formula (XXV) to obtain a compound of formula (I) wherein n is 2.

Then, the product obtained can be separated from its support and, if necessary, the protective groups of the amine and carboxyl groups can be removed.

Peptides synthesized via solid phase synthesis techniques can be cleaved and isolated according to, for example, the following non-limiting techniques: the peptide may be cleaved from the resin using techniques well known to those skilled in the art. For example, solutions of 1% or 2% trifluoroacetic acid (TFA) in dichloromethane (DCM) or a combination of a 1% and a 2% solution of TFA in DCM may be used to cleave the peptide, or a 10% solution of trifluoroethanol (TFE) in DCM may also be used. Acetic acid (AcOH), hydrochloric acid (HCl) or formic acid may also be used to cleave the peptide. In these cases the recovered peptide are fully protected except the NH in the C-terminal position. The specific cleavage reagent, solvents and time required for cleavage will depend on the particular peptide being cleaved.

Higher concentrations of acids or strongly acidic conditions such as HF in the case of for example carbonate Merrifield resin may be used to also remove the protecting. It is advantageous to use scavengers, such as silanes or other scavenger cocktails (especially for cation- and/or acid-sensitive side chain (or group) incorporating peptides), to prevent any side reactions.

After cleavage the cleavage fractions are subjected to standard work-up procedures to isolate the peptide. Typically, the combined cleavage fractions are concentrated under vacuum, followed by reconstitution with polar aprotic or polar aprotic solvents such as ethanol, methanol, isopropyl alcohol, acetone, acetonitrile, dimethyl formamide, dichloromethane, etc., followed by precipitation or crystallization with antisolvent such as water, diethyl-ether or hexanes, and collection by vacuum filtration. Alternatively, the product may be triturated with organic solvents or water after isolation of the peptide.

### PHARMACEUTICAL COMPOSITIONS

Compounds of the invention, or a pharmaceutically acceptable salt thereof, will normally, but not necessarily, be formulated into pharmaceutical compositions prior to administration to a patient.

Accordingly, in another aspect the invention is directed to pharmaceutical compositions comprising a compound of the invention, or a pharmaceutically acceptable salt thereof, and one or more pharmaceutically acceptable excipients.

These compositions can be used either as vaccine adjuvants or as non-specific stimulants of anti-infectious and anti-tumoral immunity.

Pharmaceutical compositions comprising a compound of the invention, or a pharmaceutically acceptable salt thereof, may be prepared using techniques and methods known to those skilled in the art. Some of the methods commonly used in the art are described in Remington's Pharmaceutical Sciences (Mack Publishing Company).

Used as vaccine adjuvants, the products according to the invention can be administered at the same time and by the same method as the antigen (viral, bacterial, parasitic or other antigen) against which it is desired to increase the cell immunity reactions (delayed-type hypersensitivity) or the production of circulating or local antibodies in the immunized subject (man or domestic animal).

The products are administered in relatively low doses (of the order of a mg) as a mixture with the antigen and by the same method (intramuscular, subcutaneous, intravenous, intranasal or oral method). If necessary, the product and the antigen can be emulsified in an appropriate oily excipient or incorporated into liposomes.

The dose of the compounds of the invention used in the treatment of the aforementioned disorders will vary in the usual way with the seriousness of the disorders, the weight of the sufferer, and other similar factors. However, as a general guide suitable unit doses may e lspero be 0.05 to 1000 mg, more suitably 1.0 to 500mg or 1.0 to 200 mg, and such unit doses may be administered more than once a day, for example two or three times a day.

It will be appreciated that it may be necessary to make routine variations to the dosage, depending on the age and condition of the patient and the precise dosage will be ultimately at the discretion of the attendant physician or veterinarian. The dosage will also depend on the route of administration and the particular compound selected.

As non-specific immunostimulants, the compounds of the invention are administered at doses of between 0.1 and 50 mg/kg by the parenteral method (intravenous, subcutaneous or intramuscular method) or by the intranasal, oral, rectal or, if appropriate, intra-tumoral method.

A pharmaceutical composition of a compound of the invention, or a pharmaceutically acceptable salt thereof, may be formulated for administration by any appropriate route, for example by the inhaled, nasal, oral (including buccal or sublingual), topical (including buccal, sublingual, transdermal, epicutaneous) or parenteral (subcutaneous, intramuscular, intravenous, intradermal) route.

Thus, a pharmaceutical composition of a compound of the invention, or a pharmaceutically acceptable salt thereof, may be formulated as, for example, a solution or suspension (aqueous or non-aqueous), tablet, capsule, powder, granule, lozenge, lotion, cream, ointment, gel, foam or reconstitutable powder depending on the particular route of administration.

Such pharmaceutical compositions may be prepared by any method known in the art of pharmacy, for example by bringing into association the active ingredient with the excipient(s).

Tablets and capsules for oral administration may be in unit dose presentation form, and may contain conventional excipients such as binding agents, for example syrup, acacia, gelatine, sorbitol, tragacanth, or polyvinylpyrrolidone; fillers, for example lactose, sugar, maize-starch, calcium phosphate, sorbitol or glycine; tabletting lubricants, for example magnesium stearate, talc, polyethylene glycol or silica; disintegrants, for example potato starch; or acceptable wetting agents such as sodium lauryl sulfate. The tablets may be coated according to methods well known in normal pharmaceutical practice.

Oral liquid preparations may be in the form of, for example, aqueous or oily suspensions, solutions, emulsions, syrups or elixirs, or may be presented as a dry product for reconstitution with water or other suitable vehicle before use. Such liquid preparations may contain conventional additives, such as suspending agents, for example sorbitol, methyl cellulose, glucose syrup, gelatine, hydroxyethyl cellulose, carboxymethyl cellulose, aluminium stearate gel or hydrogenated edible fats, emulsifying agents, for example lecithin, sorbitan monooleate, or acacia; non-aqueous vehicles (which may include edible oils), for example almond oil, oily esters such as glycerine, propylene glycol, or ethyl alcohol; preservatives, for example methyl or propyl hydroxybenzoate or sorbic acid, and, if desired, conventional flavouring or colouring agents.

Pharmaceutical compositions of a compound of the invention or a pharmaceutically acceptable salt thereof, for topical administration, may be presented as, for instance, ointments, creams or lotions, eye ointments and eye or ear drops, impregnated dressings and aerosols, and may contain appropriate conventional additives such as preservatives, solvents to assist drug penetration and emollients in ointments and creams. The compositions may also contain compatible conventional carriers, such as cream or ointment bases and ethanol or oleyl alcohol for lotions. Such carriers may be present as from about 1% up to about 98% of the composition. More usually they will form up to about 80% of the composition.

The compositions for parenteral administration can be suspensions, emulsions or aqueous sterile solutions. Polyethylene glycol, propylene glycol, vegetable oils, in particular olive oil, and injectable organic esters, e.g. ethyl oleate, can be employed as the vehicle in the former cases. These compositions can also contain adjuvants, in particular wetting, emulsifying or dispersing agents.

Sterilization can be carried out in several ways, e.g. using a bacteriological filter, by incorporating sterilizing agents into the composition or by heating. The compositions can also be prepared in the form of solid compositions sterilized e.g. by irradiation, which can be dissolved in sterile water or dispersed in any other injectable sterile medium, if appropriate at the time of use.

The compositions for intranasal administration can be suspensions, emulsions or aqueous sterile solutions, which can be associated, if appropriate, with a compatible propellant.

The compositions for rectal administration are suppositories which can contain excipients, such as cacao butter or a semi-synthetic glyceride, in addition to the active product.

Pharmaceutical compositions adapted for parenteral administration include aqueous and nonaqueous sterile injection solutions which may contain antioxidants, buffers, bacteriostats and solutes which render the composition isotonic with the blood of the intended recipient; and aqueous and non-aqueous sterile suspensions which may include suspending agents and thickening agents. The compositions may be presented in unit-dose or multi-dose containers, for example sealed ampoules and vials, and may be stored in a freeze-dried (lyophilized) condition requiring only the addition of the sterile liquid carrier, for example water for injections, immediately prior to use.

Extemporaneous injection solutions and suspensions may be prepared from sterile powders, granules and tablets.

Pharmaceutical compositions for topical administration to the lung may include aerosol compositions and dry powder compositions.

### COMBINATION THERAPIES

Compounds of the invention and pharmaceutically acceptable salts thereof may be used in combination preparations. For example, the compounds of formula (I) and pharmaceutically acceptable salts thereof may be used in combination with one or more compounds with activity in inflammation ; one or more compounds with activity in reducing cancer.

Examples of antiinflammatory compounds that may be used in conjunction with the compounds of the invention include antibodies and inhibitors of IL-17, IL-17R, IL-23, IL-1, TNF-alpha, VLA4 or JAK/STAT receptors.

In one embodiment, the combination as herein above defined comprises an antibody selected from Secukinumab (AIN457), Ixekizumab (LY2439821), Brodalumab (AMG-827), Ustekinumab; Canakinumab or Infliximab.

In one embodiment, the combination as herein above defined comprises an inhibitor of JAK3 and JAK1 such for example Tofacitinib.

In a further embodiment, the combination as herein above defined comprises an TNF-α antagonist such as Etanercept.

Dosages of conventional anti-tumor agents are often kept as low as possible because side effects may be observed at higher dosages.

According to the invention, a combination of NOD1 and/or agents that increase NOD1 expression or activity, with available anti-tumor agents may improve the spectrum of cancers against which those anti-tumor agents are effective and reduce the required dosage of those anti-tumor agents. Thus, the invention contemplates combinations of the present NOD1-related agents with one or more anti-tumor or carcinostatic agents.

Any anti-tumor and carcinostatic agent available to one of skill in the art can be used with the present NOD1-related agents. However, in some embodiments, the selected anti-tumor or carcinostatic agents have different mechanisms of actions, or operate against somewhat different types of cancers or tumors.

For example, the NOD1-related agents of the invention can be combined with a carcinostatic agent or an immune activator to combine the pro-apoptotic effects of NOD1 with the anti-neoplastic effect of the carcinostatis agent and/or the pro-immune responses induced by the immune activator. Further, in some cases, radiotherapy or surgical treatment is performed in addition to these methods to improve the effect of the treatment.

Examples of other chemotherapeutic agents that may be used in conjunction with the compounds of the invention include Altretamine, Bleomycin, Busulphan, Calcium Folinate, Capecitabine, Carboplatin, Carmustine, Chlorambucil, Cisplatin, Cladribine, Crisantaspase, Cyclophosphamide, Cytarabine, Dacarbazine, Dactinomycin, Daunorubicin, Docetaxel, Doxorubicin, Epirubicin, Etoposide, Fludarabine, Fluorouracil, Gemcitabine, Hydroxyurea, Idarubicin, Ifosfamide, Irinotecan, Liposomal doxorubicin, Lomustine, Melphalan, Mercaptopurine, Methotrexate, Mitomycin, Mitoxantrone, Oxaliplatin, Paclitaxel, Pentostatin, Procarbazine, Raltitrexed, Streptozocin, Tegafur-uracil, Temozolomide, Thiotepa, Tioguanine/Thioguanine, Topotecan, Treosulfan, Vinblastine, Vincristine, Vindesine, Vinorelbine and a combination thereof.

In some embodiments, the compounds of the invention are administered with one or more hormones. For example, the NOD1-related agents can be administered with one or more androgens, progesterones, estrogens or anti-estrogens. Anti-estrogens act by exerting antagonistic effects on cells or tissues that are responsive to estrogen, or by competing with estrogens for access to receptor sites located on the cell surface. For example, the drugs tamoxifen (brand name: Nolvadex) or Arimidex (Anastrozole), are anti-estrogens that can be used. Tamoxifen has been used in the treatment of breast cancer and to reduce the breast cancer incidence in high-risk women.

The invention thus provides, in a further aspect, a combination comprising a compound of the invention together with a further therapeutic agent or agents.

The combinations referred to above may conveniently be presented for use in the form of a pharmaceutical formulation and thus pharmaceutical formulations comprising a combination as defined above together with a pharmaceutically acceptable carrier or excipient comprise a further aspect of the invention. The individual components of such combinations may be administered either sequentially or simultaneously in separate or combined pharmaceutical formulations.

When a compound of the invention is used in combination with a second therapeutic agent active against the same disease state the dose of each compound may differ from that when the compound is used alone. Appropriate doses will be readily appreciated by those skilled in the art.

### UTILITY

Compounds of the invention and their pharmaceutically acceptable salts are NOD modulators, particularly they are NOD1.

Furthermore c compounds of the invention provide agonism of NOD1 and NOD2.

Thus, compounds of the invention and its pharmaceutically acceptable salts are useful in the treatment of conditions for which agonism of NOD1 or NOD1 and NOD2 receptor is beneficial.

The invention further provides a method of treatment for which agonism of NOD1 or NOD1 and NOD2 receptor is beneficial. in mammals including humans, which comprises administering to the sufferer a therapeutically effective amount of a compound of the invention or a pharmaceutically acceptable salt thereof.

In a further aspect, the present invention provides a compound of the invention and its pharmaceutically acceptable salts, for use in therapy.

Compounds of the invention or pharmaceutically acceptable salts thereof may be of use in the treatment and preventing tumor growth in a mammal, inflammation and/or apoptosis and in the treatment of infectious diseases.

In a yet further aspect, the present invention is directed to a method of treatment of an cancer disease mediated by NOD1 or NODland NOD2 which comprises administering to a subject in need thereof, a safe and therapeutically effective amount of a compound of the invention, or a pharmaceutically acceptable salt thereof.
Compounds of the invention or pharmaceutically acceptable salts thereof may be of use in the treatment of of infectious diseases.

In a further aspect, the present invention provides a compound of the invention and its pharmaceutically acceptable salts, for use in the treatment of infectious diseases.

In a yet further aspect, the present invention is directed to a method of treatment of infectious diseases mediated by NOD1 or NOD1 and NOD2 which comprises administering to a subject in need thereof, a safe and therapeutically effective amount of a compound of the invention, or a pharmaceutically acceptable salt thereof.

Compounds of the invention or pharmaceutically acceptable salts thereof may be of use in the treatment of inflammatory and/or autoimmune diseases.

Example of inflammatory and or autoimmune diseases include asthma, chronic obstructive pulmonary disease (COPD) and bronchitis, allergic diseases, such as allergic rhinitis and atopic dermatitis, cystic fibrosis, lung allograph rejection, multiple sclerosis, rheumatoid arthritis, juvenile rheumatoid arthritis, osteoarthritis, ankylosing spondylitis, systemic lupus erythematosus, psoriasis, Hashimoto's disease, pancreatisis, autoimmune diabetes, autoimmune ocular disease, ulcerative colitis, Crohn's disease, inflammatory bowel disease (IBS), inflammatory bowel syndrome (IBD), Sjorgen's syndrome, optic neuritis, type I diabetes, neuromyelitis optica, Myasthenia Gravis, uveitis, Guillain-Barre syndrome, psoriatic arthritis, Graves' disease and scleritis.

In one embodiment, compounds of the invention or pharmaceutically acceptable salts thereof may be of use in the treatment of inflammatory bowel diseases or multiple sclerosis,

In a further aspect, the present invention provides a compound of the invention or its pharmaceutically acceptable salts, for use in the treatment of cancer of infectious diseases inflammatory and or autoimmune diseases.

In a further aspect, the present invention is directed to a method of treatment of inflammatory and/or autoimmune diseases mediated by NOD1 or NOD1 and NOD2 which comprises administering to a subject in need thereof, a safe and therapeutically effective amount of a compound of the invention, or a pharmaceutically acceptable salt thereof.

### EXPERIMENTAL

The following Intermediates and Examples illustrate the preparation of compounds of the invention.
In the procedures that follow, after each starting material, reference to a description is typically provided. This is provided merely for assistance to the skilled chemist. The starting material may not necessarily have been prepared from the batch referred to.
The yields were calculated assuming that products were 100% pure if not stated otherwise.

### Abbreviations

The following abbreviations are used in the text

| | | | |
|---|---|---|---|
| °C | degree Celsius | mg | milligrams |
| µg/mL | micrograms per milliners | mg/kg | Milligrams/kilograms |
| µL | microliters | MgSO₄ | magnesium sulfate |
| Aziz | aziridine | MHz | megahertz |
| BF₃.OEt₂ | Boron trifluoride diethyl etherate | min | Minutes |
| Bn | benzyl | mL | milliliters |
| Boc | tert-butyloxy carbonyl | mmol | millimoles |
| Br | brome | MS | mass spectrometry |
| C₁₂ | Lauroyl or dodecanoyl | MurNAc | N-Acetyl muramic acid |
| C₁₄ | Myristoyl or tetradecanoyl | Na₂SO₄ | sodium sulfate |
| C₇ | Enantic or heptanoyl | NaHCO₃ | sodium hydrogen carbonate |
| CBr₄ | carbon tetrabromide | NH₄OH | ammonium hydroxide |
| CD₃OD | deuterated methanol | nm | nanometers |
| CDCl₃ | for deuterated chloroform | NMM | N-4-methylmorpholine |
| d | doublet | NMR | nuclear magnetic resonance |
| D₂O | Deuterium oxide | OD | optical density |
| DCM | dichloromethane | OMe | methoxy |
| DMF | N,N-dimethyl formamide | OxaDAP | (2S, 6R)-2,6-diamino-4-oxa-pimelic acid or meso- 2,6-diamino-4-oxa-pimelic acid |
| DMSO-*d₆* | deuterated dimethylsulfoxide | Pd/C | Palladium on activated |

| | | | |
|---|---|---|---|
| | | | charcoal |
| EDT | 1,2-ethanedithiol | Phe | phenylalanine |
| EDCI or EDC | 1-(3-diethylaminopropyl)-3-ethylcarbodiimide hydrochloride | Pht | Phtalimide |
| ES | electrospray ionization | pNZ | para nitro benzyloxy carbonyl |
| Et₂O | diethyl ether | PPh₃ | triphenyl phosphine |
| EtOAc | ethyl acetate | ppm | parts per million |
| EtOH | ethanol | PyBOP | Benzotriazole-1-yl-oxy-tris-pyrrolidino-phosphonium hexafluorophosphate |
| Fmoc | Fluorenylmethyloxycarbonyl | q | quartet |
| g | grams | rt | room temperature |
| ¹H | proton | s | singlet |
| h | hours | sc | subcutaneous |
| H₂O | water | SEAP | Secreted embryonic alkaline phosphatase |
| H₂SO₄ | sulphuric acid | Ser | serine |
| HATU | O-benzotriazol-1-yl-N,N,N',N'-tetramethyluronium hexafluorophosphate | sl | large singlet |
| HCl | hydrochloric acid | t | triplet |
| HEK | Human Embryonic Kidney Cells | THF | tetrahydrofuran |
| Hz | Hertz | TEA | triethylamine |
| | | | |
| iE-LAN | D-Glu-γ-*meso*LANOH | | |
| | | | |
| Lac | Lactoyl or 2-hydroxypropanoyl | TFA | trifluoroacetic acid |
| meso Lan m | *meso*-lanthionine multiplet | TIPS TLC | triisopropoyl silane thin layer chromatography |
| M | molar | Tr | trityl |
| m/z | mass-to-charge ratio | UV | ultra violet |
| MeOH | methanol | Z | benzyloxy carbonyl |
| | | δ | Chemical shift |

Amino acids are designated herein using standard 1-letter or three-letter codes, e.g. as designated in Standard ST.25 in the Handbook on Industrial Property Information and Documentation. The abbreviations for the α-amino acids used in this application are set forth in Table 1.Table 1

| **Amino Acid** | **3-letter Amino acid code** | **1 letter Amino acid code** |
|---|---|---|
| Alanine | Ala | A |
| Cysteine | Cys | C |
| Glutamic acid | Glu | E |
| Glycine | Gly | G |
| Leucine | Leu | L |
| Isoleucine | Ile | I |
| Valine | Val | V |
| Phenylalanine | Phe | F |
| Serine | Ser | S |
| Lanthionine | LAN | - |
| 2,6-diamino pimelic acid | DAP | - |
| 4-oxa-(2, 6)-diamino pimelic acid | OxaDAP | - |

NMR spectra were recorded on a Brucker 300 spectrometer. For 1H (300 MHz) spectra δ values were referenced to CDCl₃ (7.26 ppm), CD₃OD (3.30 ppm), or DMSO-*d₆* (2.50 ppm).

Mass spectral analyses were carried out using an Agilent 6130 MSD simple quadrupole mass spectrometer.

Protected amino acids were purchased from Novabiochem® or Iris Biotech GMBH. All commercially available reagents were used without further purification. All the solvents used for reactions were distilled over appropriate drying reagents prior to use. Commercially available ACS grade solvents (> 99.0% purity) were used for column chromatography without any further purification.

The Example disclosed herein have been named using the naming convention provided with Chem-Draw Ultra 11.0 software, available in Chem. Office.

All reactions and fractions from column chromatography were monitored by thin layer chromatography (TLC) using glass plates with a UV fluorescent indicator (normal SiO2, Merck 60 F254). One or more of the following methods were used for visualization: UV absorption by fluorescence quenching; (Ninhydrin/Ethanol or H₂SO₄/Ethanol) solution. Flash chromatography was performed using Merck type 60, 230-400mesh silica gel. IonS3 exchange chromatography was carried out using Biorad AG 50W-X8 hydrogen form resin.

### Intermediate 6

### Fmoc-Cys(Tr)OMe

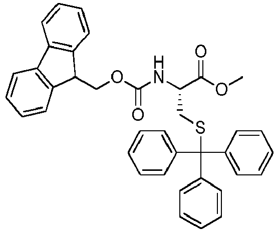

Fmoc-Cys(Tr)OH (3.0 g, 5.12 mmol) was dissolved in dry DMF (20 mL), then cesium carbonate (830 mg, 2.55 mmol) was added, the mixture was stirred at rt for 15min, then the mixture was cooled to 0°C and methyl iodide (727 mg, 5.12 mmol) was added dropwise. The stirring was maintained for 18h. Then, the solvent was removed in vacuo and the residue was diluted with EtOAc (50 mL) and the organic layer was washed with water and brine dried over MgSO₄, filtered and concentrated. The crude product was purified by flash column chromatography (silica gel, DCM 100%) to give 2.84 g (4.74 mmol, 92% yield) of the title compound. ¹H NMR (CDCl₃-*d₁*) δ 7.72 (2H, d), 7.62 (2H, d), 7.43 (4H, m), 7.33-7.20 (15H, m), 5.24 (1H, d) 4.38 (3H, m), 4.24 (1H, m), 3.74 (3H, s), 2.68 (2H, d).

### Intermediate 7

### Fmoc-CysOMe

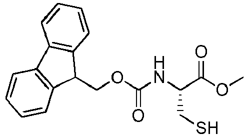

The intermediate **6** (1.8 g, 3.01 mmol) was dissolved in TFA/DCM mixture (1/1) (30 mL) and TIPS (1.57 g, 9.90 mmol) was added. After stirring for 4h at rt, the solvents were removed under vacuo, coevaporated with toluene. The compound was triturated with a mixture of petroleum ether/Et₂O (2/1) the precipitate was filtrated to provide 1.06 g (2.96 mmol, 98% yield) of the title compound. NMR (CDCl₃-*d₁*) δ 7.78 (2H, d), 7.65 (2H, d), 7.41-7.30 (4H, m), 5.70 (1H, d) 4.70 (1H, m), 4.45 (2H, m), 4.26 (1H, t), 3.78 (3H, s), 3.02 (2H, d).

### Intermediate 8

### BocD-Ala(Br)OMe

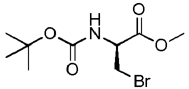

The Boc-D-SerOMe (4.43 g, 20.2 mmol) and CBr₄ (11.0 g, 33.23 mmol) were dissolved under argon in dry DCM (100 mL) and cooled to 0°C. To the stirred solution was added PPh₃ (11.0 g, 41.98 mmol) in several portions within 1h. The solution was allowed to warm to rt and stirred overnight. The solvent was diluted with DCM, washed twice with saturated NaHCO₃, water and brine. The organic layer was dried over MgSO₄, filtered and evaporated. The crude compound was purified by flash column chromatography column (silica gel, Petroleum ether/DCM 7/3 to 100% of DCM) to give 4.12 g (14.60 mmol, 72% yield) of the title compound. ¹H NMR (DMSO-*d₆*) δ 7.63 (1H, d), 4.42 (1H, m), 3.92-3.56 (2H, m), 3.68 (3H, s), 1.43 (9H, s).

### Intermediate 9

### Fmoc-mesoLAN(Boc, OMe)OMe

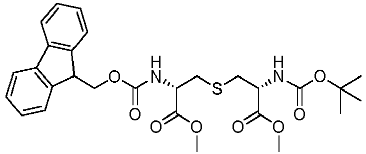

To a solution of intermediate **7** (970 mg, 2.71 mmol) and bromoserine derivative **8** (924 mg, 2.85 mmol) in EtOAc (30 mL) a pH 8.5 solution of NaHCO₃ (30 mL) was added, followed by addition of TBAHS (3.70 g, 10.91 mmol). The mixture was vigorously stirred at rt overnight. Then, the mixture was diluted with EtOAc (100 mL) and was washed successively with saturated NaHCO₃, water and brine. The organic layer was dried over MgSO₄, filtered and concentrated in vacuo. The residue was purified by flash column chromatography (silica gel, DCM/MeOH 0 to 2%) to give 1.20 g (2.00 mmol, 74% yield) of the title compound. ¹H NMR (DMSO-*d₆*) δ 7.90 (2H, d), 7.72 (2H, d), 7.45-7.31 (4H, m), 4.33-4.24 (4H, m), 3.96 (1H, m), 3.68 (3H, s), 3.66 (3H, s), 3.00-2.68 (4H, m), 1.45 (9H, s).

### Intermediate 10

### H-mesoLAN(Boc, OMe)OMe

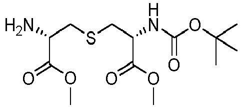

Intermediate **9** (503 mg, 0.84 mmol) was dissolved with DMF (10 mL), piperidine (15 mg, 0.16 mmol) was added dropwise , and the mixture was heated at 50°C for 1h. Then, water (25 mL) was added, and the mixture was extracted with EtOAc three times. The organic layer was washed with water (3 times) and brine, dried over MgSO₄, filtered and concentrated in vacuo. The crude compound was purified by flash column chromatography (silica gel, DCM/MeOH 0 to 5%) to provide 222 mg (0.58 mmol, 69% yield) of the title compound. ¹H NMR (DMSO-*d₆*) δ 4.18 (1H, m), 3.63 (3H, s), 3.61 (3H, s), 3.53 (1H, t), 2.82-2.73 (4H, m), 1.38 (9H, s).

### Intermediate 16

### Boc-D-Glu(OBn)OMe

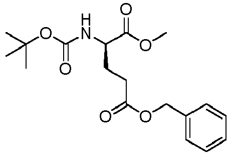

Boc-D-_{Υ}Glu(OBn)OH (5.0 g, 14.84 mmol) was dissolved in dry DMF (50 mL), then potassium carbonate (2.32 g, 16.81 mmol) was added, the mixture was stirred at rt for 15 min, then the mixture was cooled to 0°C and methyl iodide (4.21 g, 29.64 mmol) was added dropwise. The stirring was maintained for 18h. Then, the solvent was removed in vacuo and the residue was diluted with EtOAc (50 mL) and the organic layer was washed with water and brine dried over MgSO₄, filtered and concentrated. The crude product was purified by flash column chromatography (silica gel, DCM/MeOH 0 to 1%) to give 5.12 g (14.58 mmol, 98% yield) of the title compound. ¹H NMR (DMSO-*d₆*) δ 7.38-7.31 (5H, m), 7.30 (1H, d), 5.09 (2H, s), 4.00 (1H, m), 3.62 (3H, s), 2.49 (2H, m), 1.99 (1H, m), 1.81 (1H, m), 1.38 (9H,s).

### Intermediate 17

### TFA. D-Glu(OBn)OMe

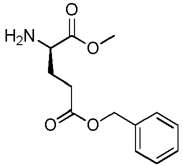

Intermediate **16** (5.1 g, 14.52 mmol) was dissolved in TFA/DCM mixture (3/1) (20 mL). After stirring for 2h at rt, the solvents were removed under vacuo; the residue was coevaporated 3 times with toluene. The crude compound was triturated with Et₂O to provide 5.19 g (14.22 mmol, 98% yield) of the title compound. ¹H NMR (DMSO-*d₆*) δ 8.45 (2H, s), 7.39-7.36 (5H, m), 5.11 (2H, s), 4.10 (1H, m), 3.62 (3H, s), 2.65 (2H, m), 2.07 (2H, m).

### Intermediate 57

### Boc-D-AlaOMe

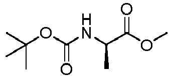

Boc-D-AlaOH (10.0 g, 52.9 mmol) was dissolved in dry DMF (60 mL), then potassium carbonate (8.0 g, 58.0 mmol) was added, the mixture was stirred at rt for 15 min, and methyl iodide (6.58 mL, 106.0 mmol) was added dropwise. The stirring was maintained for 18h. Then, the mixture was concentrated in vacuo and the residue was dissolved in EtOAc (100mL). The organic layer was washed with water, brine, dried over MgSO₄, filtered and concentrated. The crude product was purified by flash column chromatography (silica gel, DCM/MeOH 0 to 1%) to give 10.36 g (51.0 mmol, 96% yield) of the title compound. ¹H NMR (DMSO-*d₆*) δ 7.46 (1H, d), 4.35 (1H, m), 3.66 (3H, s), 1.30 (9H, s), 1.18 (3H, d).

### Intermediate 58

### TFA.D-AlaOMe

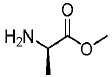

The compound **57** (10.36 g, 51.0 mmol) was dissolved in TFA/DCM mixture (1/1) (100 mL). After stirring for 1h at rt, the solvents were removed under vacuo; the residue was coevaporated 3 times with toluene. The crude compound was triturated with Et₂O to provide 11.0 g (51.0 mmol, 99% yield) of the compound. This compound was used for the next step without any further purification.

### Intermediate 59

### Fmoc-Cys(Tr)-D-AlaOMe

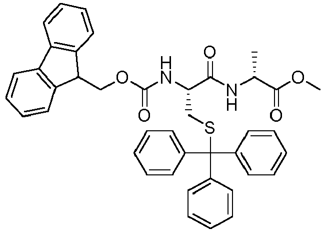

To a solution of intermediate **58** (5.6 g, 26.0 mmol) in dry DMF (120 mL) were added Fmoc-Cys(Tr)OH (10.0 g, 17.1 mmol) and HATU (7.10 g, 19.0 mmol), the pH was adjusted to 10 with NMM (6.98 mL, 68.3mmol). The reaction mixture was stirred at rt under argon for 16h. Then the reaction was concentrated in vacuo, the residue was dissolved in EtOAc (200 mL), and washed twice with a saturated solution of ammonium chloride. The organic layer was dried over MgSO₄, filtrated and concentrated in vacuo. The crude product was purified by flash column chromatography (silica gel, DCM/MeOH 0 to 1%) to give 6.17 g (9.20 mmol, 54% yield) of the title compound. ¹H NMR (DMSO-*d₆*) δ 8.28 (1H, d), 7.74 (2H, d), 7.63 (2H, d), 7.41 (1H, d), 7.33 (19H, m), 4.21-4.11 (5H, m), 3.57 (3H, s), 2.36 (2H, m), 1.23 (3H, d).

### Intermediate 60

### Fmoc-Cys-D-AlaOMe

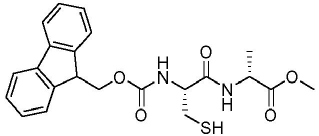

The intermediate **59** (6.17 g, 9.20 mmol) was dissolved in TFA/DCM mixture (1/1) (200 mL) and TIPS (6.22 mL, 30.35 mmol) was added. After stirring for 2h at rt, the solvents were removed under vacuo, coevaporated with toluene. The compound was triturated with a mixture of petroleum ether/Et₂O (2/1) the precipitate was filtrated to provide 3.42 g (7.98 mmol, 87% yield) of the title compound. ¹H NMR (DMSO-*d₆*) δ 8.48 (1H, d), 7.90 (2H, d), 7.74 (2H, d), 7.45 (1H, d), 7.40 (2H, m), 7.30 (2H, m), 4.32-4.16 (5H, m), 3.62 (3H, s), 2.80-2.65 (2H, m), 2.27 (1H, t), 1.27 (3H, d).

### Intermediate 61

### Fmoc-mesoLAN(Boc, OMe)-D-AlaOMe

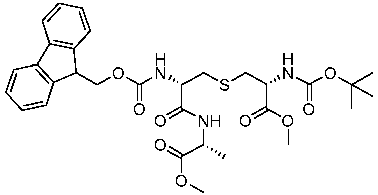

To a solution of intermediate **60** (3.42 g, 7.98 mmol) and intermediate 8 (2.5 g, 8.80 mmol) in EtOAc (100 mL) was added a pH 8.5 solution of NaHCO₃ (100 mL) followed by addition of TBAHS (11.0 g, 32.0 mmol). The mixture was vigorously stirred at rt overnight. Then, the mixture was diluted with EtOAc (200 mL) and was washed successively with saturated NaHCO₃, water and brine. The organic layer was dried over MgSO₄, filtered and concentrated in vacuo. The residue was purified by flash column chromatography (silica gel, DCM/MeOH 0 to 2%) to give 3.3 g (5.20 mmol, 66% yield) of the title compound. ¹H NMR (DMSO-*d₆*) δ 8.12 (1H, d), 7.90 (2H, d), 7.75 (2H, d), 7.60 (1H, d), 7.42 (2H, m), 7.30 (2H, m), 4.31-4.22 (6H, m), 2.81 (4H, m), 1.37 (9H, s), 1.28 (3H, d).

### Intermediate 62

### H-mesoLAN(Boc, OMe)-D-AlaOMe

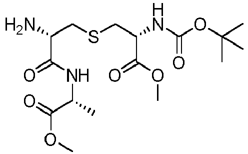

The intermediate **61** (3.3 g, 5.2 mmol) was dissolved with DMF (10 mL), piperidine (103 µL, 1.04 mmol) was added dropwise, and the mixture was heated at 50°C for 1h. Then, water (10 mL) was added, and the mixture was extracted with EtOAc three times. The organic layer was washed with water (3 times) and brine, dried over MgSO₄, filtered and concentrated in vacuo. The crude compound was purified by flash column chromatography (silica gel, DCM/MeOH 2 to 5%) to provide 1.46 g (3.57 mmol, 68% yield) of the title compound. ¹H NMR (DMSO-*d₆*) δ 8.31 (1H, d), 7.34 (1H, d), 4.30 (1H, m), 4.13 (1H, m), 3.62 (6H, s), 2.92-2.53 (4H, m), 1.90 (2H, sl), 1.45 (9H, s), 1.21 (3H, d).

### Intermediate 76

### BocGlyOMe

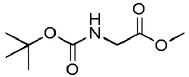

Boc-GlyOH (2.0 g, 11.41 mmol) was dissolved in dry DMF (20 mL), then potassium carbonate (1.71 g, 12.31 mmol) was added, the mixture was stirred at rt for 15 min, then the mixture was cooled to 0°C and methyl iodide (3.24 g, 22.83 mmol) was added dropwise. The stirring was maintained for 18h. Then, the solvent was removed in vacuo and the residue was diluted with EtOAc (50 mL) and the organic layer was washed with water and brine dried over MgSO₄, filtered and concentrated. The crude product was purified by flash column chromatography (silica gel, DCM/MeOH 0 to 1%) to give 2.06 g (10.88 mmol, 95% yield) of the title compound. ¹H NMR (DMSO-*d₆*) δ 7.19 (1H, m), 3.74 (2H, d), 3.65 (3H, s), 1.39 (9H, s).

### Intermediate 78

### TFA.GlyOMe

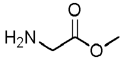

Intermediate **76** (2.059 g, 10.88 mmol) was dissolved in TFA/DCM mixture (1/3) (20 mL). After stirring for 4h at rt, the solvents were removed under vacuo; the residue was coevaporated 3 times with toluene. The crude compound was triturated with Et₂O to provide 2.21 g (10.88 mmol, 100% yield) of the title compound. ¹H NMR (DMSO-*d₆*) δ 7.19 (1H, m), 3.74 (2H, d), 3.65 (3H, s), 1.39 (9H, s).

### Intermediate 80

### Fmoc-Cys(Tr)-GlyOMe

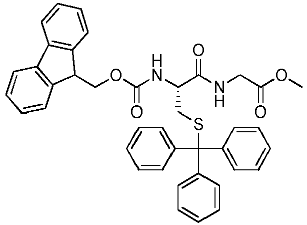

To a solution of intermediate **78** (2.00 g, 9.85 mmol) in dry DMF (120 mL) were added Fmoc-Cys(Tr)OH (4.00 g, 6.83 mmol) HATU (2.82 g, 7.42 mmol) and NMM (2.76, 27.32 mmol). The reaction mixture was stirred at rt under argon for 16h. Then the solvent of the reaction was removed in vacuo and the residue was dissolved in EtOAc (150 mL), and washed with a saturated solution of NaHCO₃, water and brine. The organic layer was dried over MgSO₄, filtrated and concentrated in vacuo. The crude product was purified by flash column chromatography (silica gel, DCM/MeOH 0 to 1%) to give 3.09 g (4.71 mmol, 69% yield) of the title compound. ¹H NMR (DMSO-*d₆*) δ 8.23 (1H, t), 7.88 (2H, d), 7.75 (2H, d), 7.45-7.22 (19H, m), 4.24 (3H, m), 4.12 (1H, m), 3.76 (2H, d), 3.55 (3H, s), 2.48 (2H, d).

### Intermediate 82

### Fmoc-Cys-GlyOBn

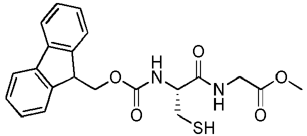

Intermediate **80** (3.09 g, 4.71 mmol) was dissolved in TFA/DCM mixture (1/1) (60 mL) and TIPS (2.46 g, 15.53 mmol) was added. After stirring for 2h at rt, the solvents were removed under vacuo, coevaporated with toluene. The compound was triturated with a mixture of petroleum the precipitate was filtrated to provide 1.79 g (4.33 mmol, 92% yield) of the the title compound. ¹H NMR (DMSO-*d₆*) δ 8.53 (1H, m), 7.91 (2H, d), 7.76 (2H, d), 7.44-7.21 (4H, m), 4.27 (4H, m), 3.88 (2H, d), 3.63 (3H, s), 2.71 (2H, m), 2.35 (1H, t).

### Intermediate 84

### Fmoc-mesoLan(Boc, OMe)-GlyOMe

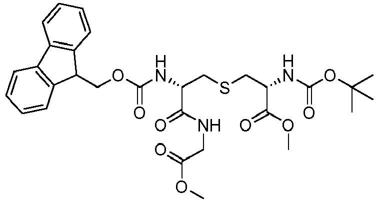

To a solution of intermediate **82** (1.79 g, 4.32 mmol) and intermediate 8 (1.51 g, 5.2 mmol) in EtOAc (60 mL) was added a pH 8.5 solution of NaHCO₃ (60 mL) followed by addition of TBAHS (5.93 g, 17.49 mmol). The mixture was vigorously stirred at rt overnight. Then, the mixture was diluted with EtOAc (200 mL) and was decanted then the organic layer was washed successively with saturated NaHCO₃, water and brine. The organic layer was dried over MgSO₄, filtered and concentrated in vacuo. The residue was purified by flash column chromatography (silica gel, DCM/MeOH 0 to 2%) to give 2.18 g (3.54 mmol, 82% yield) of the title compound. ¹H NMR (DMSO-*d₆*) δ 8.52 (1H, m), 7.90 (2H, d), 7.74 (2H, d), 7.45-7.30 (4H, m), 4.24 (4H, m), 3.87 (2H, d), 3.63 (3H, s), 2.88-2.61 (4H, m), 1.37 (9H,s).

### Intermediate 86

### H-mesoLan(Boc, OMe)-GlyOMe

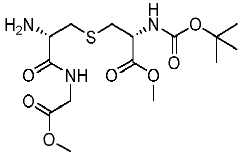

Intermediate **84** (2.18 g, 3.54 mmol) was dissolved with DMF (20 mL), piperidine (60 mg, 0.71 mmol) was added dropwise , and the mixture was heated at 50°C for 45 min. Then, water (20 mL) was added, and the mixture was extracted with EtOAc 3 times. The organic layer was washed with water (3 times) and brine, dried over MgSO₄, filtered and concentrated in vacuo. The crude compound was purified by flash column chromatography (silica gel, DCM/MeOH 2% to 5%) to provide 0.857 g (2.18 mmol, 61% yield) of the title compound. ¹HNMR (DMSO-*d₆*) δ 8.45 (1H, m), 7.30 (1H, d), 4.13 (1H, m), 3.86 (2H, d), 3.64 (3H, s), 3.63 (3H,s), 3.32 (1H, m), 2.89-2.60 (4H, m), 1.39 (9H,s).

m), 4.25 (2H, m), 3.83 (2H, d), 3.61 (1H, m), 2.88-2.66 (4H, m), 2.16 (2H, m), 2.01 (1H, m), 1.80 (1H, m).

### Intermediate 103

### Fmoc-Ala-D-Glu(OBn)OMe

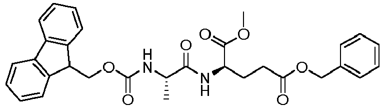

To a solution of intermediate **17** (4.40 g, 12.0 mmol) in dry DMF (50 mL) were added intermediate Fmoc-AlaOH (3.0 g, 9.60 mmol) HATU (4.0 g, 11.0 mmol) and NMM (9.78 mL, 19.3 mmol). The reaction mixture was stirred at rt under argon for 16h. Then the solvent of the reaction was removed in vacuo and the residue was dissolved in EtOAc (150 mL), and washed with a saturated solution of NaHCO₃, water and brine. The organic layer was dried over MgSO₄, filtrated and concentrated in vacuo. The crude product was purified by flash column chromatography (silica gel, DCM/MeOH 0 to 2%) to give 5.11 g (9.38 mmol, 97% yield) of the title compound. ¹H NMR (DMSO-*d₆*) δ 8.29 (1H, d), 7.88 (2H, d), 7.73 (2H, m), 7.50 (1H, d), 7.33 (9H, m), 5.06 (2H, s), 4.26 (4H, m), 4.22 (1H, m), 3.61 (3H, s), 2.41 (2H, m), 2.03 (1H, m), 1.89 (1H, m), 1.21 (3H, d).

### Intermediate 104

### Fmoc-Ala-D-GluOMe

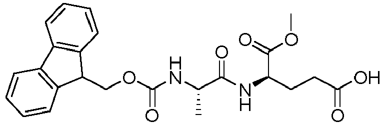

Intermediate **103** (2.0 g, 3.7 mmol) was dissolved in a mixture of THF/MeOH mixture (1/1) (40 mL) Nickel de Ranay (215 mg) was added and the mixture was stirred under hydrogen atmosphere for 2h. Then, the mixture was filtered over Celite, the celite was washed with MeOH. The filtrate was concentrated in vacuo to give 406 mg (0.89 mmol, 40% yield) of the title compound. This compound was used for the next step without any further purification. ¹H NMR (DMSO-*d₆*) δ 8.28 (1H, d), 7.88 (2H, d), 7.73 (2H, m), 7.39 (5H, m), 4.27 (4H, m), 4.12 (1H, m), 3.62 (3H, s), 2.25 (2H, m), 1.95 (1H, m), 1.79 (1H, m), 1.22 (3H, d).

### Intermediate 105

### Fmoc-Ala-D-GluOMe-γ-mesoLan(Boc, OMe)OMe

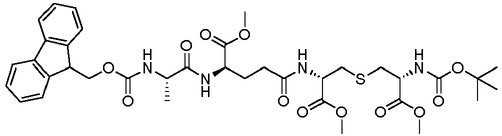

To a solution of intermediate 104 (150 mg, 0.33 mmol) in dry DMF (10 mL) were added intermediate **10** (128 mg, 0.38 mmol) HATU (140 mg, 0.36 mmol) and NMM (37 µL, 0.36 mmol). The reaction mixture was stirred at rt under argon for 16h. Then the solvent of the reaction was removed in vacuo and the residue was dissolved in EtOAc (30 mL), and washed with a saturated solution of NaHCO₃, water and brine. The organic layer was dried over MgSO₄, filtrated and concentrated in vacuo. The crude product was purified by flash column chromatography (silica gel, DCM/MeOH 0 to 2%) to give 202 mg (0.26 mmol, 79% yield) of the title compound. ¹H NMR (DMSO-*d₆*) δ 8.76 (1H, d), 8.60 (1H, d), 8.38 (1H, d), 8.31 (1H, d), 7.89 (2H, d), 7.73 (2H, t), 7.51 (2H, m), 7.44 (2H, t), 7.30 (3H, m), 4.43 (1H, m), 4.24 (3H, m), 4.09 (2H, m), 3.62 (6H, s), 3.62 (9H, s), 2.88-2.70 (4H, m), 2.21 (2H, t), 2.10 (2H, t), 1.96 (1H, m), 1.80 (1H, m), 1.37 (9H, m), 1.23 (3H, d).

### Intermediate 106

### Fmoc-Ala-D-GluOMe-γ-mesoLan(Boc, OMe)-GlyOMe

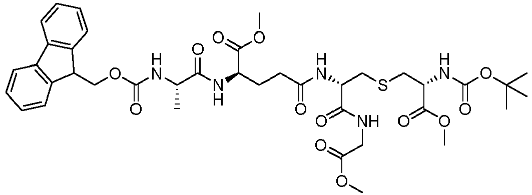

To a solution of intermediate 104 (120 mg, 0.26 mmol) in dry DMF (10 mL) were added intermediate **86** (120 mg, 0.30 mmol) HATU (110 mg, 0.29 mmol) and NMM (27 µL, 0.26 mmol). The reaction mixture was stirred at rt under argon for 16h. Then the solvent of the reaction was removed in vacuo and the residue was dissolved in EtOAc (30 mL), and washed with a saturated solution of NaHCO₃, water and brine. The organic layer was dried over MgSO₄, filtrated and concentrated in vacuo. The crude product was purified by flash column chromatography (silica gel, DCM/MeOH 0 to 2%) to give 150 mg (0.18 mmol, 68% yield) of the title compound. ¹H NMR (CDCl₃-*d₁*) δ 7.78 (2H, d), 7.62 (2H, d), 7.40 (2H, t), 7.31 (2H, t), 4.75 (1H, m), 4.60 (1H, m), 4.47 (2H, m), 4.28 (2H, m), 4.05 (2H, m), 3.62 (6H, s), 3.61 (3H, s), 2.96-2.80 (4H, m), 2.34 (2H, m), 1.84 (2H, m), 1.45 (12H, m).

### Intermediate 107

### Fmoc-Ala-D-GluOMe-γ-mesoLan(Boc, OMe)-D-AlaOMe

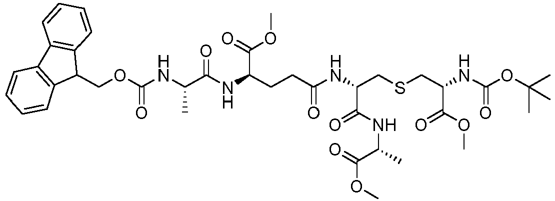

To a solution of intermediate 104 (155 mg, 0.38 mmol) in dry DMF (10 mL) were added intermediate **62** (150 mg, 0.33 mmol) HATU (140 mg, 0.36 mmol) and NMM (37 µL, 0.36 mmol). The reaction mixture was stirred at rt under argon for 16h. Then the solvent of the reaction was removed in vacuo and the residue was dissolved in EtOAc (30 mL), and washed with a saturated solution of NaHCO₃, water and brine. The organic layer was dried over MgSO₄, filtrated and concentrated in vacuo. The crude product was purified by flash column chromatography (silica gel, DCM/MeOH 0 to 2%) to give 200 mg (0.24 mmol, 72% yield) of the title compound. ¹H NMR (DMSO-*d₆*) δ 8.53 (1H, d), 8.30 (1H, d), 8.12 (1H, d), 7.88 (2H, m), 7.73 (2H, m), 7.49-7.26 (m, 6H), 4.50 (1H, m), 4.25 (5H, m), 4.12 (2H, m), 3.61 (9H, s), 2.88-2.73 (4H, m), 2.20 (2H, m), 1.95 (1H, m), 1.79 (1H, m), 1.28 (9H, m), 1.22 (6H, m).

### Intermediate 108

### H-Ala-D-GluOMe-γ-mesoLan(Boc, OMe)OMe

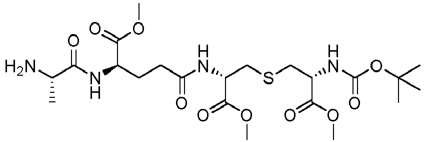

Intermediate **105** (202 mg, 0.26 mmol) was dissolved with DMF (10 mL), piperidine (5 µL, 0.05 mmol) was added. The mixture was heated at 50°C for 45 min. Then, water (20 mL) was added, and the mixture was extracted with EtOAc 3 times. The organic layer was washed with water (3 times) and brine, dried over MgSO₄, filtered and concentrated in vacuo. The crude compound was purified by flash column chromatography (silica gel, DCM/MeOH 5% to 10%) to provide 54 mg (0.09 mmol, 37% yield) of the title compound. ¹HNMR (MeOD-*d₁*) δ 8.65 (1H, d), 8.33 (1H, d), 7.45 (1H, m), 4.66 (1H, m), 4.49 (1H, m), 4.46 (1H, m), 4.0.1 (1H, m), 3.65 (9H, s), 3.18-2.87 (4H, m), 2.40 (2H, m), 2.25 (1H, m), 2.00 (1H, m), 1.55 (3H, d), 1.46 (9H, s).

### Intermediate 109

### H-Ala-D-GluOMe-γ-mesoLan(Boc, OMe)-GlyOMe

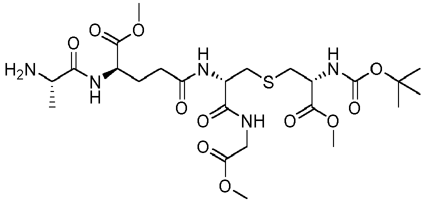

Intermediate **106** (150 mg, 0.18 mmol) was dissolved with DMF (10 mL), piperidine (3µL, 0.03 mmol) was added. The mixture was heated at 50°C for 45 min. Then, water (20 mL) was added, and the mixture was extracted with EtOAc 3 times. The organic layer was washed with water (3 times) and brine, dried over MgSO₄, filtered and concentrated in vacuo. The crude compound was purified by flash column chromatography (silica gel, DCM/MeOH 2% to 5%) to provide 74 mg (0.12 mmol, 67% yield) of the title compound. ¹HNMR (DMSO-*d₆*) δ 8.65 (1H, d), 8.33 (1H, d), 7.45 (1H, m), 4.66 (1H, m), 4.49 (1H, m), 4.46 (1H, m), 3.86 (2H, d), 3.65 (9H, s), 3.18-2.87 (4H, m), 2.40 (2H, m), 2.25 (1H, m), 2.01 (1H, m), 1.54 (3H, d), 1.47 (9H, s).

### Intermediate 110

### H-Ala-D-GluOMe-γ-mesoLan(Boc, OMe)-D-AlaOMe

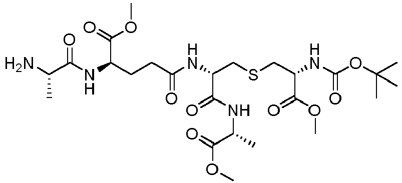

Intermediate **107** (200 mg, 0.24 mmol) was dissolved with DMF (10 mL), piperidine (5µL, 0.04 mmol) was added. The mixture was heated at 50°C for 45 min. Then, water (20 mL) was added, and the mixture was extracted with EtOAc 3 times. The organic layer was washed with water (3 times) and brine, dried over MgSO₄, filtered and concentrated in vacuo. The crude compound was purified by flash column chromatography (silica gel, DCM/MeOH 2% to 5%) to provide 101 mg (0.16 mmol, 69% yield) of the title compound. ¹HNMR (MeOD) δ 4.61 (1H, m), 4.49 (2H, m), 4.36 (1H, m), 3.74 (9H, s), 3.50 (1H, m), 3.04-2.76 (4H, m), 2.40 (2H, m), 2.28 (1H, m), 1.95 (1H, m), 1.46 (9H, d), 1.35 (3H, d), 1.19 (3H, d).

### Intermediate 112

### 1-OBn-4,6-0-Benzylidene-MurNAc-Ala-D-Glu(OBn)Ome-γ-mesoLAN(Boc, OMe)OMe

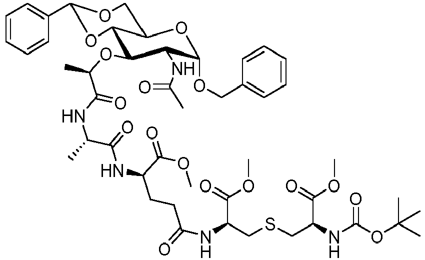

Benzyl-2-acetamido-4,6-0-dibenzylidene-2-deoxy-3-0-[(R)-1-carboxyethyl]-α-D-glucopyranoside (106 mg, 0.22 mmol; prepared by the process set forth in Gross et al., Liebigs Ann. Chem., 37-45 (1986)) was suspended in dry DMF (4 mL), then were added compound **108** (124 mg, 0.22 mmol) HATU (94 mg, 0.25 mmol) and NMM (24 µL, 0.23 mmol). The reaction mixture was stirred at rt under argon for 2h. Then the solvent of the reaction was removed in vacuo and the residue was dissolved in EtOAc (15 mL), and washed with a saturated solution of NaHCO₃, water and brine. The organic layer was dried over MgSO₄, filtrated and concentrated in vacuo. The crude product was purified by flash column chromatography (silica gel, DCM/MeOH 0 to 2%) to give 129 mg (0.13 mmol, 57% yield) of the title compound. ¹H NMR (DMSO-*d₆*) δ 8.44 (1H, d), 8.39 (1H, d), 7.55 (1H, d), 7.38 (11H, m), 5.70 (1H, s), 4.88 (1H, d), 4.48 (2H, m), 4.44-4.19 (6H, m), 3.97 (1H, m), 3.73 (4H, m), 3.62 (6H, s), 3.60 (3H, s), 2.88-2.73 (4H, m), 2.20 (2H, t), 2.08 (1H, m), 1.79 (4H, m), 1.37 (9H, s), 1.21 (6H, t).

### Intermediate 113

### 1-OBn-4,6-0-Benzylidene-MurNAc-Ala-D-GluOMe-γ-mesoLAN(Boc, OMe)-GlyOMe

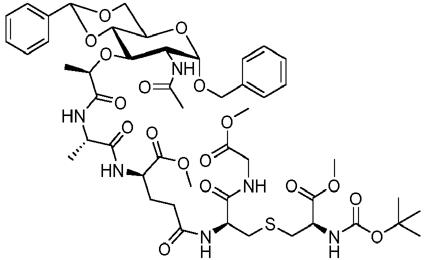

Benzyl-2-acetamido-4,6-0-dibenzylidene-2-deoxy-3-0-[(R)-1-carboxyethyl]-α-D-glucopyranoside (44 mg, 0.09 mmol was suspended in dry DMF (5 mL), then were added compound **109** (56 mg, 0.09 mmol) HATU (39 mg, 0.10 mmol) and NMM (10 µL, 0.10 mmol). The reaction mixture was stirred at rt under argon for 2h. Then the solvent of the reaction was removed in vacuo and the residue was dissolved in EtOAc (15 mL), and was filtrated, washed with Et₂O and dried in vacuo to give 72 mg (0.06 mmol, 72% yield) of the title compound. ¹H NMR (DMSO-*d₆*) δ 8.46 (2H, d), 8.18 (2H, d), 7.35 (1H, d), 7.32 (11H, m), 5.70 (1H, s), 4.87 (1H, d), 4.48 (3H, m), 4.38-4.28 (5H, m), 4.01 (1H, m), 3.85 (2H, d), 3.72 (4H, m), 3.62 (3H, s), 3.61 (3H, s), 3.60 (3H, s), 2.83-2.72 (4H, m), 2.17 (2H, t), 2.08 (2H, m), 1.79 (3H, s), 1.37 (9H, s), 1.21 (6H, t).

### Intermediate 114

### 1-OBn-4,6-0-Benzylidene-MurNAc-Ala-D-GluOMe-γ-mesoLAN(Boc, OMe)-D-AlaOMe

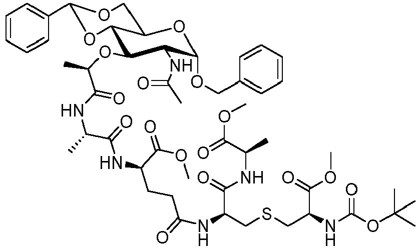

Benzyl-2-acetamido-4,6-0-dibenzylidene-2-deoxy-3-0-[(R)-1-carboxyethyl]-α-D-glucopyranoside (65 mg, 0.14 mmol was suspended in dry DMF (5 mL), then were added compound **110** (98 mg, 0.16 mmol) HATU (58 mg, 0.15 mmol) and NMM (42 µL, 0.41 mmol). The reaction mixture was stirred at rt under argon for 2h. Then the solvent of the reaction was removed in vacuo and the residue was dissolved in EtOAc (15 mL), and was filtrated, washed with Et₂O and dried in vacuo to give 95 mg (0.09 mmol, 65% yield) of the title compound. ¹H NMR (DMSO-*d₆*) δ 8.55 (1H, d), 8.45 (1H, d),8.15 (2H, d), 7.55 (1H, d), 7.30 (11H, m), 5.70 (1H, s), 4.87 (1H, d), 4.70 (1H, m),4.50 (2H, m), 4.30-4.17 (6H, m), 3.98 (1H, m), 3.85 (2H, d), 3.89 (4H, m), 3.61 (9H, s), 3.06-2.74 (4H, m), 2.27 (2H, t), 2.08 (2H, m), 1.79 (3H, s), 1.37 (9H, s), 1.22 (9H, m).

### EXAMPLES

### Example B

General protocol for cleavage of the methyl ester and tert-butyloxycarbonyl group:
To a solution of protected intermediate in dioxane (5 mL) was added a solution of lithium hydroxyde (1 M), the mixture was stirred at rt overnight or at 50°C for 2H00. Then the mixture was acidified (pH 4) by addition of Amberlite. The mixture was filtered and the resine was washed with MeOH, then the solvent was removed in vacuo and the residue was directly dissolved in TFA (5 mL). The mixture was stirred for 2H00 at RT. Then the solvents were removed under vacuo; the residue was coevaporated 3 times with toluene. The crude compound was triturated with Et₂O to provide the title compound as trifluoroacetate salt.

### Example 31

### TFA N²-(((R)-2-(((2S,3R,4R,5S,6R)-3-acetamido-2-(benzyloxy)-5-hydroxy-6-(hydroxymethyl)tetrahydro-2H-pyran-4-yl)oxy)propanoyl)-L-alanyl)-N⁵-((S)-2-(((R)-2-amino-2-carboxyethyl)thio)-1-carboxyethyl)-D-glutamine

### [1-OBn-MurNAc-Ala-D-Glu-γ-mesoLANOH (BnMurNAc-A-iE-LAN)]

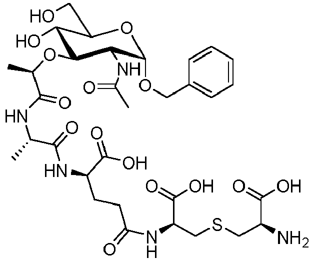

Example 31 was prepared from intermediate 112 using a similar procedure to that described in Example B, to provide 49 mg (0.06 mmol, 71% yield) of the title compound. ¹H NMR (DMSO-*d₆*) δ 8.38-8.11 (4H, m), 7.73 (1H, d), 7.35 (5H, m), 4.78 (2H, m), 4.45-4.17 (4H, m), 3.78 (1H, m), 3.63 (2H, m), 3.18-2.72 (4H, m), 2.27 (2H, m), 1.83 (1H, m), 1.78 (3H, s), 1.77 (1H, m), 1.21 (6H, m). MS (+)-ES [M+H]⁺ 774 m/z.

### Example 32

### TFA N²-(((R)-2-(((2S,3R,4R,5S,6R)-3-acetamido-2-(benzyloxy)-5-hydroxy-6-(hydroxymethyl)tetrahydro-2H-pyran-4-yl)oxy)propanoyl)-L-alanyl)-N⁵-((S)-3-(((R)-2-amino-2-carboxyethyl)thio)-1-((carboxymethyl)amino)-1-oxopropan-2-yl)-D-glutamine

### [1-OBn-MurNAc-Ala-D-Glu-γ-mesoLAN-GlyOH (BnMurNAc-A-iE-LAN-G)]

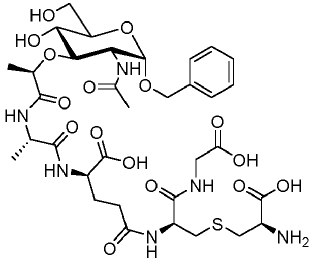

Example 32 was prepared from intermediate 113 using a similar procedure to that described in Example B, to provide 45 mg (0.05 mmol, 81% yield) of the title compound.. ¹H NMR (DMSO-*d₆*) δ 8.35-8.14 (4H, m), 7.63 (1H, d), 7.35 (5H, m), 4.81-4.10 (8H, m), 3.78 (3H, m), 3.12-2.72 (4H, m), 2.26 (2H, m), 2.08 (1H, m), 1.78 (3H, s), 1.77 (1H, m), 1.23 (6H, m). MS (+)-ES [M+H]⁺ 831 m/z.

### Example 33

### TFA N₂-(((R)-2-(((2S,3R,4R,5S,6R)-3-acetamido-2-(benzyloxy)-5-hydroxy-6-(hydroxymethyl)tetrahydro-2H-pyran-4-yl)oxy)propanoyl)-L-alanyl)-N⁵-((S)-3-(((R)-2-amino-2-carboxyethyl)thio)-1-(((R)-1-carboxyethyl)amino)-1-oxopropan-2-yl)-D-glutamine

### [1-OBn-MurNAc-Ala-D-Glu-γ-mesoLAN-D-AlaOH (BnMurNAc-A-iE-LAN-D-Ala)]

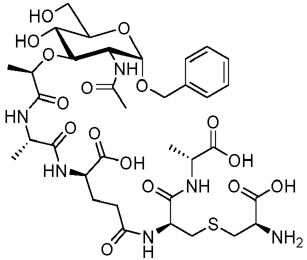

Example 33 was prepared from intermediate **114** using a similar procedure to that described in Example B, to provide 36 mg (0.04 mmol, 48% yield) of the title compound.. ¹H NMR (DMSO-*d₆*) δ 8.30-7.80 (5H, m), 7.36 (5H, m), 4.78-4.67 (2H, m), 4.42-4.27 (6H, m), 3.80 (3H, m), 3.12-2.72 (4H, m), 2.15 (2H, m), 2.00 (1H, m), 1.78 (3H, s), 1.77 (1H, m), 1.23 (9H, m). MS (+)-ES [M+H]⁺ 845 m/z.

### BIOLOGICAL DATA

The biological activities of exemplified compounds of formula (I) were assessed in the following assays:

### NOD1 functional agonist assay protocol

HEK293 cells were stably transfected with plasmid for the overexpression of the NOD1 genes and a reporter plasmid expressing a secreted embryonic alkaline phosphatase (SEAP) gene under the control of a promoter inducible by the transcription factors NF-κB and AP-1. These cells are referred herein as 293hNOD1-SEAP (for cells overexpressing the human NOD1 gene) and 293mNOD1-SEAP (for cells overexpressing the murine NOD1 gene). Upon NOD1 stimulation, these cells induce the activation of NF-κB and AP-1 and subsequently the secretion of SEAP. The reporter protein is easily detectable and measurable when using QUANTI-Blue™ (InvivoGen, San Diego, CA), a medium that turns purple/blue in the presence of SEAP.

The biological activity of the compounds of the invention tested using 293hNOD1 and 293mNOD1 cells. SEAP expression was measured using the commercially available SEAP detection kit (HEK-Blue™ Detection, InvivoGen). The SEAP activity in cells was quantified using the microplate reader (FLUOStar OPTIMA, BMG laboratories), the absorbance values were read using filter with excitation at 620 nm and emission at 655 nm. The results provided in Table 2 were expressed as the concentration were a significant mean optical density (OD) can be observed (above 3*O.D of negative control). As a positive control, the NOD1 pathway was stimulated with commercially available iE-DAP (InvivoGen) and C₁₂-iE-DAP (an acylated derivative of DAP; InvivoGen). As a negative control endotoxin-free water was used. Additionally, 293 cells expressing the SEAP gene but not the NOD1 gene were used as a control.

**Table 2**

| **Examples** | **mNod1 (µg/mL)** | **hNod1 (µg/mL)** |
|---|---|---|
| **31** | 1 | 1 |
| **32** | 0.1 | 0.1 |
| **33** | 1 | 1 |

### NOD2 functional agonist assay protocol

HEK293 cells were stably transfected with plasmid for the overexpression of the NOD2 genes and a reporter plasmid expressing a secreted embryonic alkaline phosphatase (SEAP) gene under the control of a promoter inducible by the transcription factors NF-κB and AP-1. These cells are referred herein as 293hNOD2-SEAP. Upon NOD2 stimulation, these cells induce the activation of NF-κB and AP-1 and subsequently the secretion of SEAP. The reporter protein is easily detectable and measurable when using QUANTI-Blue™ (InvivoGen, San Diego, CA), a medium that turns purple/blue in the presence of SEAP.

The biological activity of the compounds using 293hNOD2 cells. SEAP expression was measured using the commercially available SEAP detection kit (HEK-Blue™ Detection, InvivoGen). The SEAP activity in cells was quantified using the microplate reader (FLUOStar OPTIMA, BMG laboratories), the absorbance values were read using filter with excitation at 620 nm and emission at 655 nm. The results provided in Table 3 were expressed as the concentration were a significant mean optical density (OD) can be observed (above 3*O.D of negative control) as the mean optical density. As a positive control, the NOD2 pathway was stimulated with commercially available MDP (InvivoGen) and M-triDAP (InvivoGen). As a negative control endotoxin-free water was used. Additionally, 293 cells expressing the SEAP gene but not the NOD2 gene were used as a control.

**Table 3**

| **Examples** | **hNod2 (µg/mL)** |
|---|---|
| **31** | 0.1 |
| **32** | 0.1 |
| **33** | 1 |

### 3. DSS-induced inflammation in the colon model

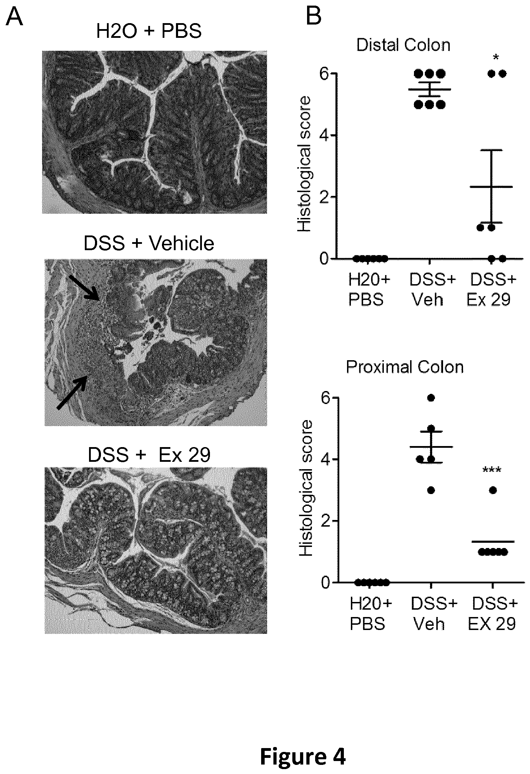

Figure 4(A) shows a representative histology from individual mice. Arrows indicate areas of severe inflammatory pathology. Figure 4(B) shows inflammatory scores for proximal and distal colon for each group of mice (mean ± SE; n=6). *, P<0.05; and ***, P<0.001 versus DSS + vehicle treated mice. The examples described in the drawing sheets 1-4 are reference examples.

## Claims

1. A compound selected from the group consisting of 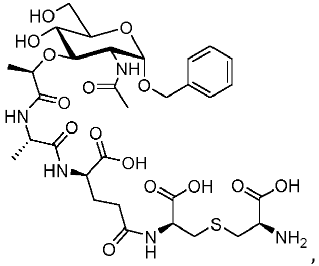 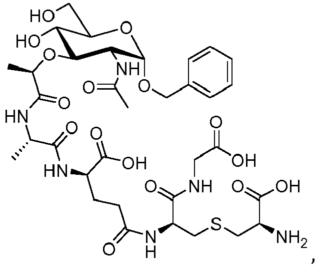 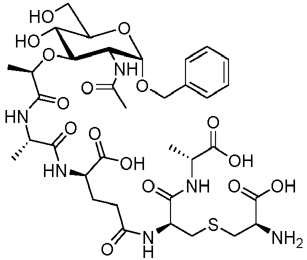 or a pharmaceutically acceptable salt thereof.

2. A compound or a pharmaceutically acceptable salt thereof, as defined in claim 1, for use in therapy.

3. A compound or a pharmaceutically acceptable salt thereof, as defined in claim 1, for use in treatment of conditions for which agonism of NOD1 or NODland NOD2 receptors is beneficial.

4. A compound as claimed in claim 1 or a pharmaceutically acceptable salt thereof for use in the treatment of patients with inflammatory and/or autoimmune diseases.

5. A compound as claimed in claim 4, wherein inflammatory and or autoimmune diseases is select from asthma, chronic obstructive pulmonary disease (COPD) and bronchitis, allergic rhinitis and atopic dermatitis, cystic fibrosis, lung allograph rejection, multiple sclerosis, rheumatoid arthritis, juvenile rheumatoid arthritis, osteoarthritis, ankylosing spondylitis, systemic lupus erythematosus, psoriasis, Hashimoto's disease, pancreatisis, autoimmune diabetes, autoimmune ocular disease, ulcerative colitis, Crohn's disease, inflammatory bowel disease (IBS), inflammatory bowel syndrome (IBD), Sjorgen's syndrome, optic neuritis, type I diabetes, neuromyelitis optica, Myasthenia Gravis, uveitis, Guillain-Barre syndrome, psoriatic arthritis, Graves' disease or scleritis.

6. A compound as claimed in claim 4 or 5, wherein inflammatory and/ or autoimmune diseases are multiple sclerosis or inflammatory bowel diseases.

7. A pharmaceutical composition comprising a) a compound defined by claim 1 or a pharmaceutically acceptable salt thereof and b) one or more pharmaceutically acceptable exicipents.

## Patentansprüche

1. Eine Verbindung, ausgewählt aus der Gruppe, bestehend aus 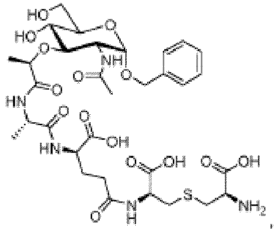 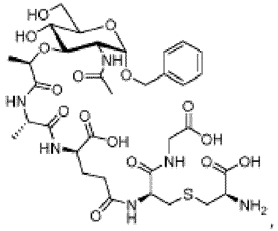 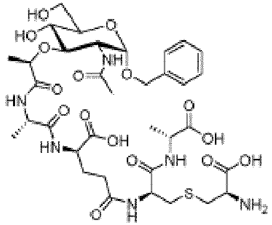 oder einem pharmazeutisch annehmbaren Salz davon.

2. Verbindung oder pharmazeutisch annehmbares Salz davon, wie in Anspruch 1 definiert, zur Verwendung bei Therapie.

3. Verbindung oder pharmazeutisch annehmbares Salz davon, wie in Anspruch 1 definiert, zur Verwendung bei der Behandlung von Krankheiten, für die ein Agonismus von NOD1- oder NOD1- und NOD2-Rezeptoren nützlich ist.

4. Verbindung gemäß Anspruch 1 oder pharmazeutisch annehmbares Salz davon zur Verwendung bei der Behandlung von Patienten mit entzündlichen und/oder Autoimmunerkrankungen.

5. Verbindung gemäß Anspruch 4, wobei die entzündlichen und/oder Autoimmunerkrankungen aus Asthma, chronisch obstruktiver Lungenerkrankung (COPD) und Bronchitis, allergischer Rhinitis und atopischer Dermatitis, Mukoviszidose, Lungenallotransplantatabstoßung, Multipler Sklerose, rheumatoider Arthritis, juveniler rheumatoider Arthritis, Osteoarthritis, Spondylitis ankylosans, systemischem Lupus erythematodes, Psoriasis, Hashimoto-Erkrankung, Pankreatitis, Autoimmundiabetes, Autoimmunaugenerkrankung, Colitis ulcerosa, Morbus Crohn, entzündlicher Darmerkrankung (IBS), Reizdarmsyndrom (IBD), Sjörgen-Syndrom, Optikusneuritis, Diabetes Typ 1, Neuromyelitis optica, Myasthenia gravis, Uveitis, Guillain-Barré-Syndrom, Psoriasis-Arthritis, Graves-Krankheit oder Skleritis ausgewählt sind.

6. Verbindung gemäß Anspruch 4 oder 5, wobei die entzündlichen und/oder Autoimmunerkrankungen Multiple Sklerose oder entzündliche Darmerkrankungen sind.

7. Eine pharmazeutisch annehmbare Zusammensetzung, beinhaltend a) eine Verbindung, definiert gemäß Anspruch 1, oder ein pharmazeutisch annehmbares Salz davon und b) einen oder mehrere pharmazeutisch annehmbare Arzneimittelträger.

## Revendications

1. Un composé sélectionné dans le groupe constitué de 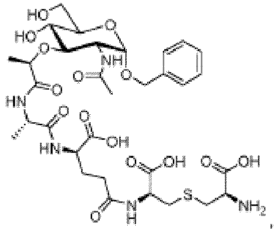 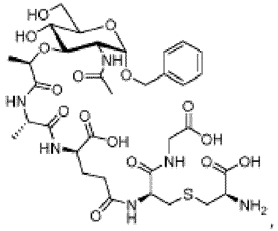 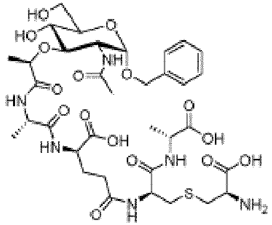 ou un sel pharmaceutiquement acceptable de celui-ci.

2. Un composé ou un sel pharmaceutiquement acceptable de celui-ci, tel que défini dans la revendication 1, pour une utilisation thérapeutique.

3. Un composé ou un sel pharmaceutiquement acceptable de celui-ci, tel que défini dans la revendication 1, pour une utilisation dans le traitement d'états pour lesquels l'agonisme du récepteur NOD1 ou des récepteurs NOD1 et NOD2 est bénéfique.

4. Un composé tel que revendiqué dans la revendication 1 ou un sel pharmaceutiquement acceptable de celui-ci pour une utilisation dans le traitement de patients atteints de maladies inflammatoires et/ou auto-immunes.

5. Un composé tel que revendiqué dans la revendication 4, dans lequel les maladies inflammatoires et/ou auto-immunes sont sélectionnées parmi asthme, bronchopneumopathie chronique obstructive (BPCO) et bronchite, rhinite allergique et dermatite atopique, mucoviscidose, rejet d'allogreffe du poumon, sclérose en plaques, polyarthrite rhumatoïde, polyarthrite rhumatoïde juvénile, arthrose, spondylarthrite ankylosante, lupus érythémateux disséminé, psoriasis, maladie de Hashimoto, pancréatite, diabète auto-immun, maladie oculaire auto-immune, rectocolite hémorragique, maladie de Crohn, maladie inflammatoire de l'intestin (MII), syndrome de l'intestin irritable (SII), syndrome de Sjögren, névrite optique, diabète de type I, neuromyélite optique, myasthénie, uvéite, syndrome de Guillain-Barré, rhumatisme psoriasique, maladie de Graves ou sclérite.

6. Un composé tel que revendiqué dans la revendication 4 ou la revendication 5, dans lequel les maladies inflammatoires et/ou auto-immunes sont la sclérose en plaques ou les maladies inflammatoires de l'intestin.

7. Une composition pharmaceutique comprenant a) un composé défini par la revendication 1 ou un sel pharmaceutiquement acceptable de celui-ci et b) un ou plusieurs excipients pharmaceutiquement acceptables.
